# EUROPEAN PATENT APPLICATION

(11) **EP 4 566 627 A1**
(43) Date of publication of application: **11.06.2025**
(21) Application number: 23846296.4
(22) Date of filing: 18.07.2023
(51) Int. Cl.: A61K 39/395, A61K 45/00, A61P 1/14, A61P 1/16, A61P 3/00, A61P 3/04, A61P 3/06, A61P 3/10, A61P 9/00, A61P 9/10, A61P 9/12, A61P 11/00, A61P 13/12, A61P 19/02, A61P 19/08

(54) **NEUROTRIMIN FUNCTION INHIBITOR**

(30) Priority: 28.07.2022 JP 2022120544
(71) Applicant: The University of Tokyo, Bunkyo-ku, Tokyo 113-8654 (JP)
(72) Inventor: AKIYAMA, Tetsu, Tokyo 113-8654 (JP); KAMOSHIDA, Yuki, Tokyo 113-8654 (JP); HAYASHI, Tomoatsu, Tokyo 113-8654 (JP); YAMAZUMI, Yusuke, Tokyo 113-8654 (JP); ODA, Takeaki, Tokyo 113-8654 (JP); SHIKANAI, Mima, Tokyo 113-8654 (JP)
(74) Representative: Vossius & Partner Patentanwälte Rechtsanwälte mbB
(86) International application number: PCT/JP2023/026213
(87) International publication number: WO 2024/024565

(57) **Abstract**

It is an object of the present invention to provide a cellular senescence-suppressing agent, and a preventive or therapeutic agent for diseases, etc. provided by cellular senescence. More specifically, the present invention relates to a cellular senescence-suppressing agent, comprising, as an active ingredient, an agent for inhibiting the function of NTM (Neurotrimin), and a medicament, etc., comprising the cellular senescence-suppressing agent. Moreover, the present invention relates to an antibody that suppresses senescence. Furthermore, the present invention relates to a method of screening for a substance that suppresses cellular senescence.

## Description

### Technical Field

The present invention relates to a Neurotrimin function inhibitor. Furthermore, the present invention relates to a cellular senescence-suppressing agent, an agent for improving individual aging and obesity, and an agent for treating various diseases induced by cellular senescence, each of which contains a Neurotrimin function inhibitor.

### Background Art

In modern society that faces super-aging, extending healthy life expectancy is one of the most important issues that modern science must address. It is clear that effective methods for extending healthy life expectancy include reforming the medical system and improving lifestyle-related diseases, dietary habits, etc. However, a fundamental response to healthy life expectancy essentially requires a comprehensive understanding of the mechanisms that control aging and the development of techniques of preventing aging-related diseases and a decline in the functions of organs and tissues, which are accompanied with aging.

Cellular senescence refers to a state in which cell proliferation has irreversibly terminated, and cellular senescence is considered to play an important role in aging at the individual level and the onset of aging-related diseases. For example, through genetic analysis using progeroid mouse models, Baker et al. have reported that when senescent cells have artificially been removed from old mice, the onset of geriatric diseases such as arteriosclerosis and renal dysfunction is significantly delayed, and further, the lifespan itself is even extended (Non Patent Literature 1). Moreover, when the influence of senescent cells on healthy life expectancy was examined in non-progeroid mice, it was suggested that accumulation of p16-positive cells, one of the biomarkers of senescent cells, in the body tends to shorten the lifespan, and that the removal of p16-positive cells is likely to extend the healthy life expectancy of an individual (Non Patent Literature 2). Therefore, if senescent cells present in the body could be selectively removed, or if cellular senescence could be suppressed or terminated, it would be considered to lead to the establishment of new methodologies for extending healthy life expectancy or treating aging-related diseases (arteriosclerosis and osteoporosis).

Cellular senescence is considered to be a chronic inflammatory state. It has been reported that this chronic inflammatory state is a cause of carcinogenesis and the occurrence of malignancy of cancer (Non Patent Literature 3). Cellular senescence had once been thought to terminate cell proliferation and to act to suppress cancer. In recent years, however, it has been shown that the aging state of cells has a dual role in cancer and may promote proliferation of cancer cells in some cases (Non Patent Literature 4).

In addition, obese state is also a chronic inflammatory state, just like cellular senescence, and it has been reported that cellular senescence also progresses under an obese state (Non Patent Literature 5). In such an obese state, inflammation and cellular senescence are particularly induced in adipose tissues (Non Patent Literature 6), and cellular senescence in these adipose tissues becomes a factor for acquisition of insulin resistance associated with obesity (Non Patent Literature 7).

It has been reported that, in aged cells (senescent cells), the expression of cellular senescence markers such as p16 (CDKN2A), p15 (CDKN2B) and p21 (CDKN1A) is increased, and at the same time, inflammatory cytokines (e.g., IL-6, IL-8, etc.), chemokines, matrix metalloproteinases, growth factors, and the like are secreted. This secretory phenomenon is called senescence-associated secretory phenotype (SASP), and it has been suggested that the senescence-associated secretory phenotype is related to the onset of aging-related diseases (Non Patent Literature 8 to Non Patent Literature 10). For example, it has been widely reported that IL-6, an inflammatory cytokine, greatly contributes to various cancers (Non Patent Literature 11). In addition, it has also been suggested that SASP-related factors may act in a paracrine manner as factors to provide an unfavorable microenvironment that promotes inflammation and carcinogenesis in tissues surrounding senescent cells (Non Patent Literature 12).

Neurotrimin (NTM) was identified as a membrane protein expressed in the developmental stage of brain (Non Patent Literature 13). NTM is one type of GPI-anchored protein that is anchored to the cell membrane by glycosylphosphatidylinositol (GPI). NTM has a signal peptide sequence necessary for extracellular secretion at the N-terminus thereof, a signal peptide sequence necessary for GPI attachment at the C-terminus thereof, and further, three IgG-like domains in the center of the molecule thereof (Non Patent Literature 14). The tissue in which NTM is most highly expressed is the brain, but the expression of NTM is also observed in other tissues, mainly, in adipose tissues, etc. Almost all researches regarding NTM have been conducted on the brain and the nervous system, and it is considered that NTM is involved in the elongation and polymerization of nerve cells (Non Patent Literature 15).

Research using NTM-deficient mice has shown that the lack of NTM leads to changes in higher brain functions such as fear behavior (Non Patent Literature 16). Although the functions of NTM in other tissues remain significantly unclear, some reports suggest the relation of NTM with cancer. For example, NTM is specifically expressed in a high level in cancer tissues, and it has been reported that antibodies targeting NTM that is immobilized on the surface of cancer cells kill the cancer cells via ADCC (antibody-dependent cell-mediated cytotoxicity) or CDC (complement-dependent cytotoxicity) (Patent Literature 1).

### Citation List

### Patent Literature

Patent Literature 1: JP Patent Publication (Kokai) No. 2010-133705 A

### Non Patent Literature

Non Patent Literature 1: Baker et al., Nature 479: 232-236, 2011.
Non Patent Literature 2: Baker et al., Nature 530: 184-189, 2016.
Non Patent Literature 3: Furman et al., Nat. Med. 25: 1822-1832, 2019.
Non Patent Literature 4: Sieben et al., Trends Cell Biol. 9: 723-737, 2018.
Non Patent Literature 5: Ferrucci et al., Nat. Rev. Cardiol. 15: 505-522, 2018.
Non Patent Literature 6: Liu et al., Clin. Sci. 134: 315-330, 2020.
Non Patent Literature 7: Minamino et al., Nat. Med. 15: 1082-1087, 2009.
Non Patent Literature 8: Birch et al., Genes Dev. 34: 1565-1576, 2020.
Non Patent Literature 9: Sharpless et al., Nat. Rev. Cancer 15: 397-408, 2015.
Non Patent Literature 10: van Deursen, Nature 509: 439-446, 2014.
Non Patent Literature 11: Johnson et al., Nat. Rev. Clin. Oncol. 15: 234-248, 2018.
Non Patent Literature 12: Coppe et al., PLoS Biol. 6: 2853-2868, 2008.
Non Patent Literature 13: Struyk et al., J. Neurosci. 15: 2141-2156, 1995.
Non Patent Literature 14: Lodge et al., Mol Cell Neurosci.17: 746-760, 2001.
Non Patent Literature 15: Gil et al., J Neurosci. Nov 18: 9312-9325. 1998.
Non Patent Literature 16: Mazitov et al., Behav Brain Res. 15: 317: 311-318, 2017.

### Summary of Invention

### Technical Problem

In view of the above-described circumstances, it is an object of the present invention to provide a cellular senescence-suppressing agent (a substance that suppresses or terminates cellular senescence). Moreover, it is another object of the present invention to provide: a medicament comprising, as an active ingredient, the cellular senescence-suppressing agent, which is an agent for improving the states provoked by senescent cells (e.g., individual aging, obesity, etc.); a medicament for treating diseases provided by senescent cells (e.g., inflammatory disease, cancer, diabetes, etc.); or the like.

### Solution to Problem

The present inventors have searched for a gene group involved in senescence by introducing an siRNA library into a system in which senescence is induced by allowing Ras to express in normal human diploid IMR-90 cells. Among the searched gene group, a Neurotrimin (NTM) gene was identified as a gene encoding a secretory protein that can be a target for antibody drugs. By knocking down the NTM gene, cellular senescence was suppressed, and the expression of the cellular senescence markers p15 and p16 and the inflammatory marker IL-6 in the cells was reduced. Furthermore, from the results of the analysis of NTM knockout mice, insulin sensitivity, and a high-fat diet loading test, it was anticipated that NTM would be involved in aging, metabolism, inflammation, and carcinogenesis.

As described above, the present inventors have revealed for the first time that NTM is involved in cellular senescence.

Furthermore, the present inventors have prepared hybridomas that produce human monoclonal antibodies against NTM, and have succeeded in obtaining multiple clones that produce antibodies suppressing the expression of p15 (a cellular senescence marker) and IL-6 (an inflammatory cytokine) in senescent-induced cells from the hybridomas.

The present invention has been completed based on the aforementioned findings.

Specifically, the present invention includes the following (1) to (15).
(1) A cellular senescence-suppressing agent, comprising, as an active ingredient, an NTM (Neurotrimin) function inhibitor.
(2) The cellular senescence-suppressing agent according to the above (1), wherein the NTM function inhibitor is an antibody, a peptide aptamer, a specialty peptide, a nucleic acid or a low-molecular-weight compound.
(3) An agent for improving individual aging, comprising the cellular senescence-suppressing agent according to the above (1) or (2).
(4) An agent for improving obesity, comprising the cellular senescence-suppressing agent according to the above (1) or (2).
(5) A pharmaceutical composition for preventing or treating diseases provoked by cellular senescence, the pharmaceutical composition comprising the cellular senescence-suppressing agent according to the above (1) or (2).
(6) The pharmaceutical composition according to the above (5), wherein the diseases are cancer, diabetes, hypertension, dyslipidemia, arteriosclerosis, cerebral infarction, heart disease (myocardial infarction, cardiomyopathy and cardiac hypertrophy), chronic obstructive pulmonary disease, chronic kidney disease, chronic liver disease (cirrhosis, hepatitis and fatty liver), dementia (Alzheimer's disease), Parkinson's disease, frailty syndrome, sarcopenia, emaciation, osteoarthritis, spondylosis deformans, skin disease, senile alopecia, cataracts, dry eyes, glaucoma, age-related macular degeneration, presbyopia, other chronic inflammatory diseases, and other metabolic diseases and/or infectious diseases.
(7) A food and drink composition or a cosmetic composition, comprising the cellular senescence-suppressing agent according to the above (1) or (2).
(8) The food and drink composition or the cosmetic composition according to the above (7), which is for use in anti-aging, dieting, or lowering blood sugar levels.
(9) An antibody, which is characterized in that the amino acid sequences of CDRs (complementarity determining regions) 1 to 3 satisfy any of the following (A), (B), (C), (D) or (E), or an antigen-binding fragment thereof:
   (A)
      heavy chain CDR1 comprising the amino acid sequence as set forth in SEQ ID No: 3,
      heavy chain CDR2 comprising the amino acid sequence as set forth in SEQ ID No: 4,
      heavy chain CDR3 comprising the amino acid sequence as set forth in SEQ ID No: 5,
      light chain CDR1 comprising the amino acid sequence as set forth in SEQ ID No: 6,
      light chain CDR2 comprising the amino acid sequence as set forth in SEQ ID No: 7, and
      light chain CDR3 comprising the amino acid sequence as set forth in SEQ ID No: 8;
   (B)
      heavy chain CDR1 comprising the amino acid sequence as set forth in SEQ ID No: 9,
      heavy chain CDR2 comprising the amino acid sequence as set forth in SEQ ID No: 10,
      heavy chain CDR3 comprising the amino acid sequence as set forth in SEQ ID No: 11,
      light chain CDR1 comprising the amino acid sequence as set forth in SEQ ID No: 12,
      light chain CDR2 comprising the amino acid sequence as set forth in SEQ ID No: 13, and
      light chain CDR3 comprising the amino acid sequence as set forth in SEQ ID No: 14;
   (C)
      heavy chain CDR1 comprising the amino acid sequence as set forth in SEQ ID No: 15,
      heavy chain CDR2 comprising the amino acid sequence as set forth in SEQ ID No: 16,
      heavy chain CDR3 comprising the amino acid sequence as set forth in SEQ ID No: 17,
      light chain CDR1 comprising the amino acid sequence as set forth in SEQ ID No: 18,
      light chain CDR2 comprising the amino acid sequence as set forth in SEQ ID No: 19, and
      light chain CDR3 comprising the amino acid sequence as set forth in SEQ ID No: 20;
   (D)
      heavy chain CDR1 comprising the amino acid sequence as set forth in SEQ ID No: 21,
      heavy chain CDR2 comprising the amino acid sequence as set forth in SEQ ID No: 22,
      heavy chain CDR3 comprising the amino acid sequence as set forth in SEQ ID No: 23,
      light chain CDR1 comprising the amino acid sequence as set forth in SEQ ID No: 24,
      light chain CDR2 comprising the amino acid sequence as set forth in SEQ ID No: 25, and
      light chain CDR3 comprising the amino acid sequence as set forth in SEQ ID No: 26; or
   (E)
      heavy chain CDR1 comprising the amino acid sequence as set forth in SEQ ID No: 27,
      heavy chain CDR2 comprising the amino acid sequence as set forth in SEQ ID No: 28,
      heavy chain CDR3 comprising the amino acid sequence as set forth in SEQ ID No: 29,
      light chain CDR1 comprising the amino acid sequence as set forth in SEQ ID No: 30,
      light chain CDR2 comprising the amino acid sequence as set forth in SEQ ID No: 31, and
      light chain CDR3 comprising the amino acid sequence as set forth in SEQ ID No: 32.
(10) An antibody that suppresses or inhibits the function of NTM, the antibody competitively inhibiting the binding between the antibody according to the above (9) and NTM, or an antigen-binding fragment thereof.
(11) The antigen-binding fragment according to the above (9) or (10), which is characterized in that it is Fab, Fab', F (ab')₂, Fv, a single-chain antibody, scFv, an scFv dimer, or dsFv.
(12) A method of screening for a substance that suppresses cellular senescence, comprising the following (a) and (b):
   (a) a step of allowing NTM and a candidate substance to come into contact with cells; and
   (b) a step of evaluating the degree of cellular senescence of the cells with which the NTM and the candidate substance are allowed to come into contact.
(13) The method according to the above (12), wherein the degree of cellular senescence is evaluated using, as an indicator, the expression level of a cellular senescence marker or the expression level of an SASP (senescence-associated secretory phenotype)-related factor.
(14) The method according to the above (13), wherein the cellular senescence marker is p16, p15, or p21.
(15) The method according to the above (13), wherein the SASP-related factor is an IL-6 or IL-8 gene.

It is to be noted that the preposition "to" used in the present description indicates a numerical value range including the numerical values located left and right of the preposition.

### Advantageous Effects of Invention

By using the agent, medicament, etc. according to the present invention, cellular senescence can be inhibited or suppressed. As a result, it becomes possible to improve the states provoked by senescent cells (e.g., individual aging, obesity, etc.), or to treat diseases provoked by senescent cells (e.g., inflammatory disease, cancer, diabetes, etc.).

### Brief Description of Drawings

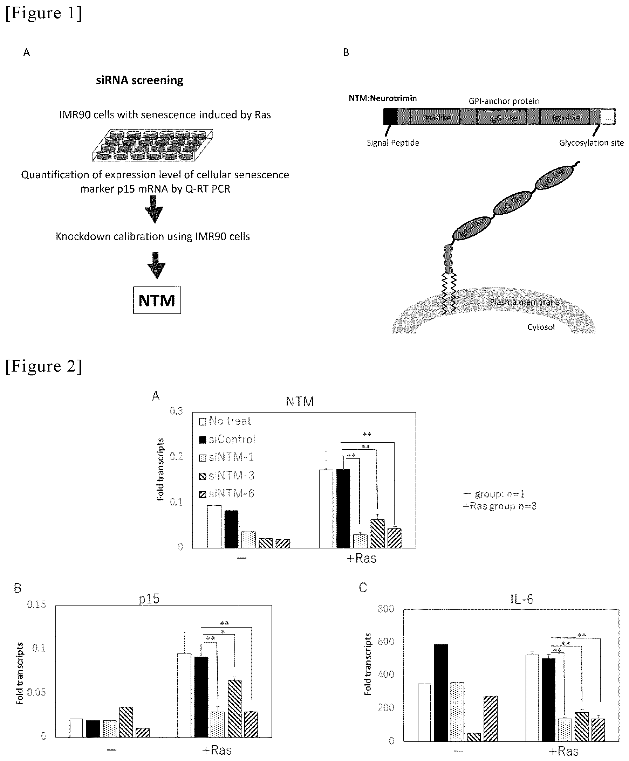

[Figure 1] Figure 1 shows an outline of a method of identifying a novel senescent gene and the structure of the identified NTM. Figure 1A shows an outline of a method of screening for a novel senescent gene. Figure 1B is a view schematically showing the structure of NTM, and a state in which NTM is anchored on the cell membrane.
[Figure 2] Figure 2 shows the results of NTM gene knockdown by siRNA. The expression levels of NTM (A), p15 (B) and IL-6 (C) in IMR90 cells in which NTM was knocked down by siRNA were measured under conditions with senescence induction by Ras or under conditions without such induction.

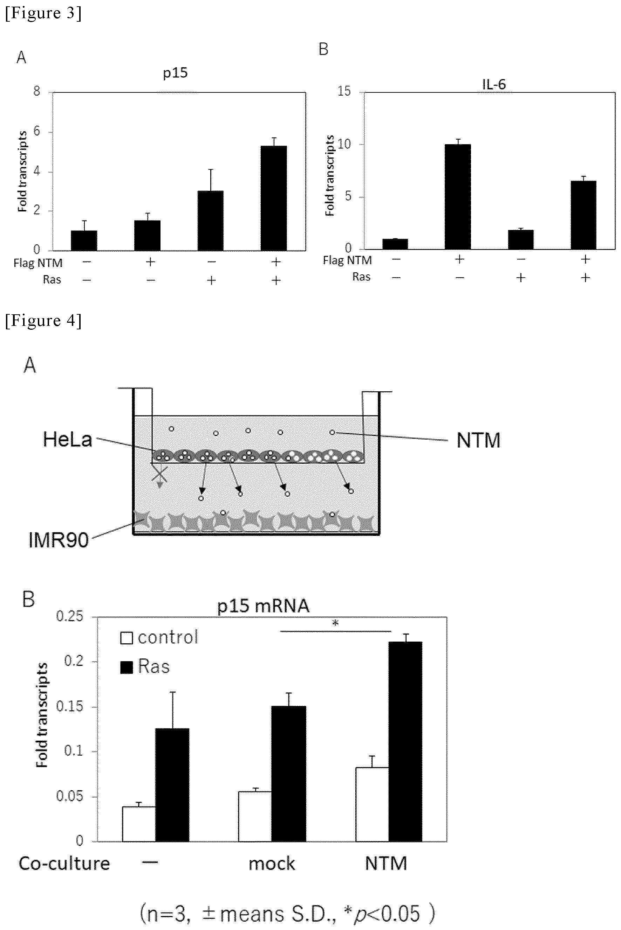

[Figure 3] Figure 3 shows the results obtained by allowing NTM to forcibly express in cells. The gene expression levels of p15 (A) and IL-6 (B) in IMR90 cells in which NTM was expressed were measured under conditions of senescence induction by Ras or under conditions without such induction.
[Figure 4] Figure 4 shows the results of functional analysis of secretory NTM. Figure 4A is a schematic view showing the state of co-culture of NTM-secreting HeLa cells and IMR90 cells. Figure 4B shows the results obtained by measuring the expression level of p15 mRNA in IMR90 cells.

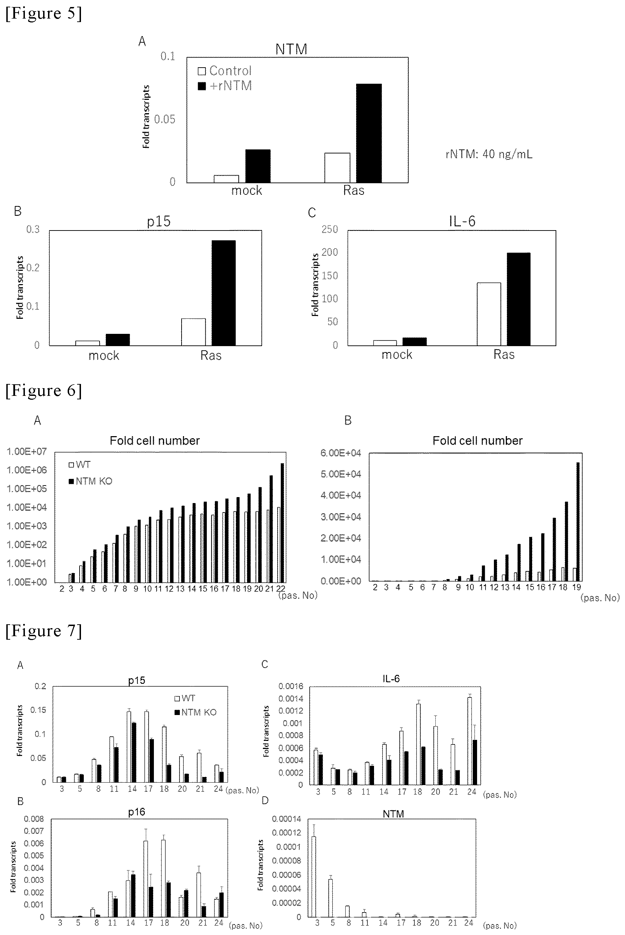

[Figure 5] Figure 5 shows the results obtained by confirming the senescence-inducing action of recombinant NTM. The gene expression levels of NTM (A), p15 (B), and IL-6 (C) were measured in IMR90 cells (Ras) with senescence induction by Ras or IMR90 cells without senescence induction (mock).
[Figure 6] Figure 6 shows the results obtained by investigating the role of NTM in the senescence of mouse MEFs (1). This figure shows the results in which MEFs derived from littermate wild-type mice (WT) and NTM-deficient mice (NTM KO) were repeatedly passaged, and the cell proliferation rate was examined at each passage.
[Figure 7] Figure 7 shows the results obtained by investigating the role of NTM in the senescence of mouse MEFs (2). This figure shows the results in which MEFs derived from littermate wild-type mice (WT) and NTM-deficient mice (NTM KO) were repeatedly passaged, and the expression levels of p15 (A), p16 (B), IL-6 (C) and NTM (D) were examined at each passage.

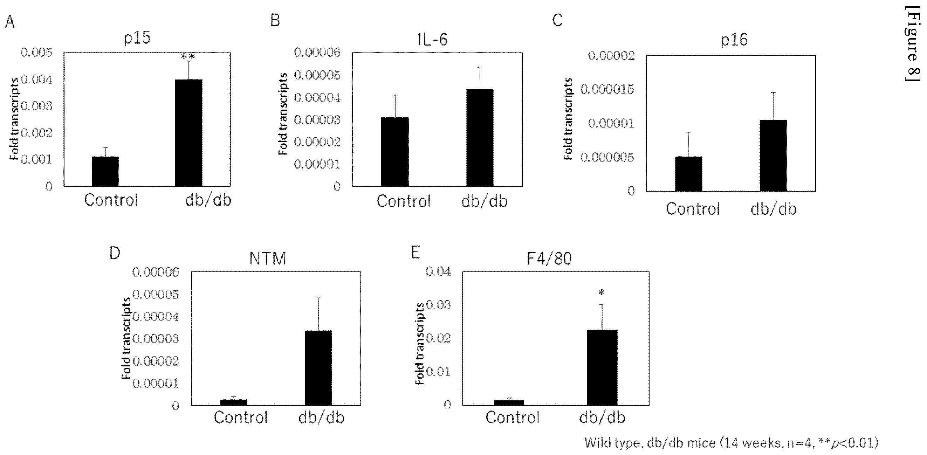

[Figure 8] Figure 8 shows the results obtained by investigating the role of NTM in obesity. The gene expression levels of NTM (D), the macrophage marker F4/80 (E), and the senescence-related factors p15 (A), IL-6 (B), and p16 (C) were measured in the adipose tissues of db/db mice that are overeating-type obesity mouse models. Control: wild-type mice; db/db: obese mouse models.

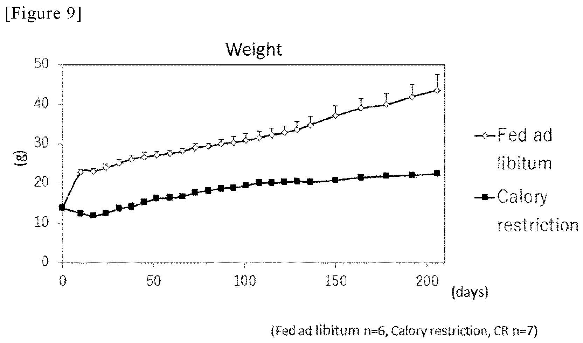

[Figure 9] Figure 9 shows the results obtained by measuring changes in the body weights of mice that underwent calorie restriction. Fed ad libitum: free-feeding mouse group; Calory restriction: calorie-restricted mouse group.

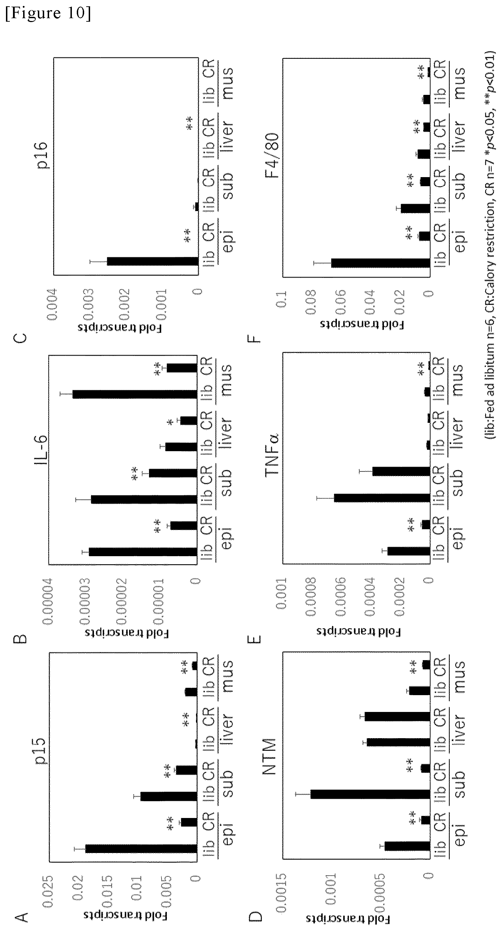

[Figure 10] Figure 10 shows the influence of calorie restriction on the expression of NTM and senescence-related factors. The gene expression levels of p15 (A), IL-6 (B), p16 (C), NTM (D), the inflammatory cytokine TNFα (E), and F4/80 (F) were measured in the metabolic control organs of free-feeding mice (lib) and calorie-restricted mice (CR). epi: visceral adipose tissues (epididymal fat); sub: subcutaneous adipose tissues (subcutaneous fat); liver: liver; mus: muscle; lib: free-feeding mouse group; CR: calorie-restricted mouse group.

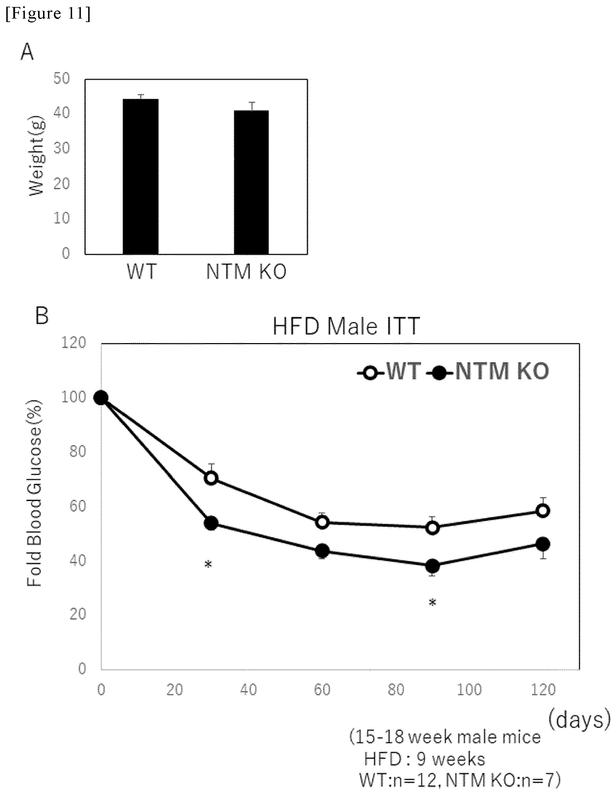

[Figure 11] Figure 11 shows the results of an analysis of NTM-deficient mice fed with a high-fat diet (1). This figure shows the results of the body weight change (A) and the insulin tolerance test (ITT) results (B) of NTM-deficient mice fed with a high-fat diet (NTM KO) and wild-type mice (WT).

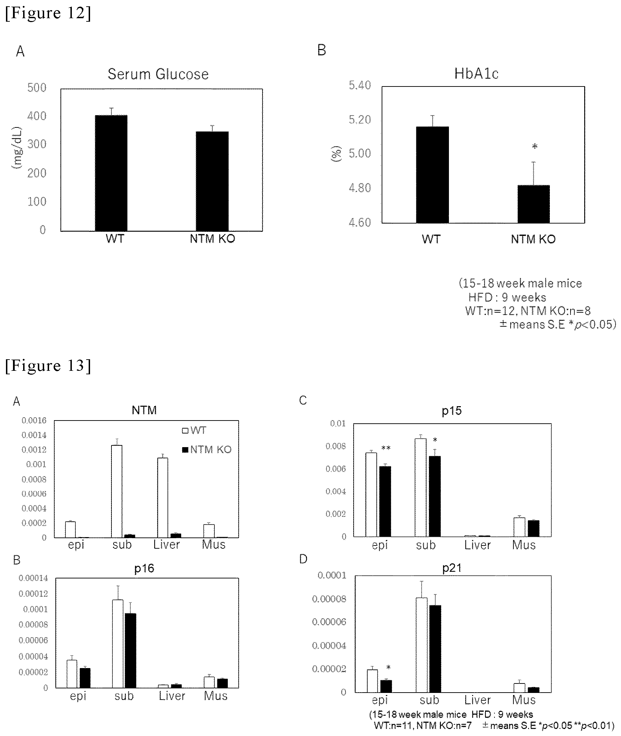

[Figure 12] Figure 12 shows the results of an analysis of NTM-deficient mice fed with a high-fat diet (2). This figure shows the results obtained by measuring the fasting blood glucose levels (A) and the HbA1c levels (B) were measured in NTM-deficient mice fed with a high-fat diet (NTM KO) and wild-type mice (WT).
[Figure 13] Figure 13 shows the results of an analysis of NTM-deficient mice fed with a high-fat diet (3). The gene expression levels of NTM (A), p16 (B), p15 (C), and p21 (D) were measured in the metabolic control organs of NTM-deficient mice fed with a high-fat diet (NTM KO) and wild-type mice (WT). epi: visceral adipose tissues; sub: subcutaneous adipose tissues liver: liver; mus: muscle.

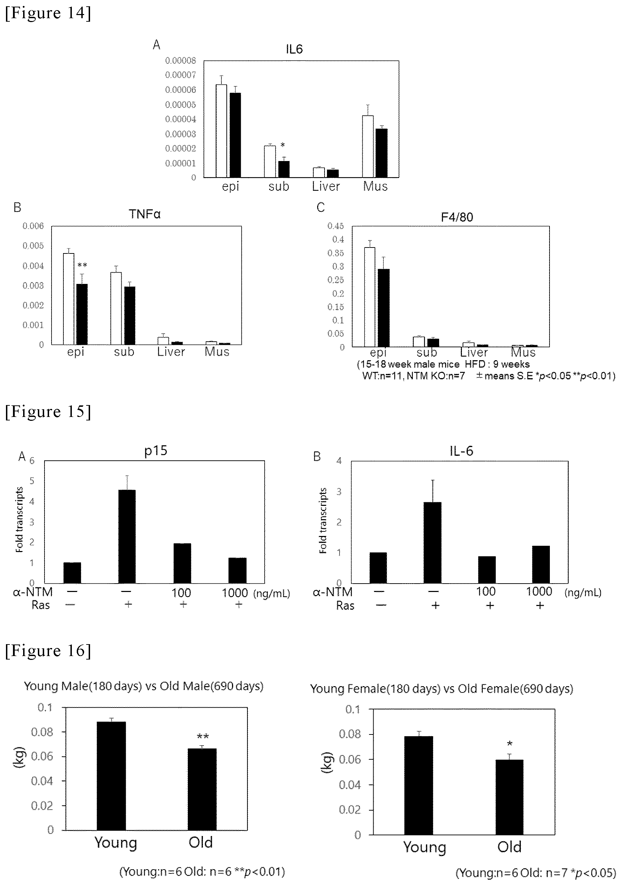

[Figure 14] Figure 14 shows the results of an analysis of NTM-deficient mice fed with a high-fat diet (4). The gene expression levels of IL6 (A), TNFα (B), and F4/80 (C) were measured in the metabolic control organs of NTM-deficient mice fed with a high-fat diet (NTM KO) and wild-type mice (WT). epi: visceral adipose tissues; sub: subcutaneous adipose tissues; liver: liver; mus: muscle.
[Figure 15] Figure 15 shows the results obtained by investigating the cellular senescence-suppressing effect by inhibition of NTM function. A fully human antibody against NTM was added to IMR90 cells in which senescence was induced by Ras, and the gene expression levels of p15 (A) and IL-6 (B) were then measured in the IMR90 cells.
[Figure 16] Figure 16 shows the results obtained by comparing muscle strength between young mice and old mice. The left figure shows the results of comparing muscle strength between wild-type young male mice (Young) and old male mice (Old), whereas the right figure shows the results of comparing muscle strength between wild-type young female mice (Young) and old female mice (Old).

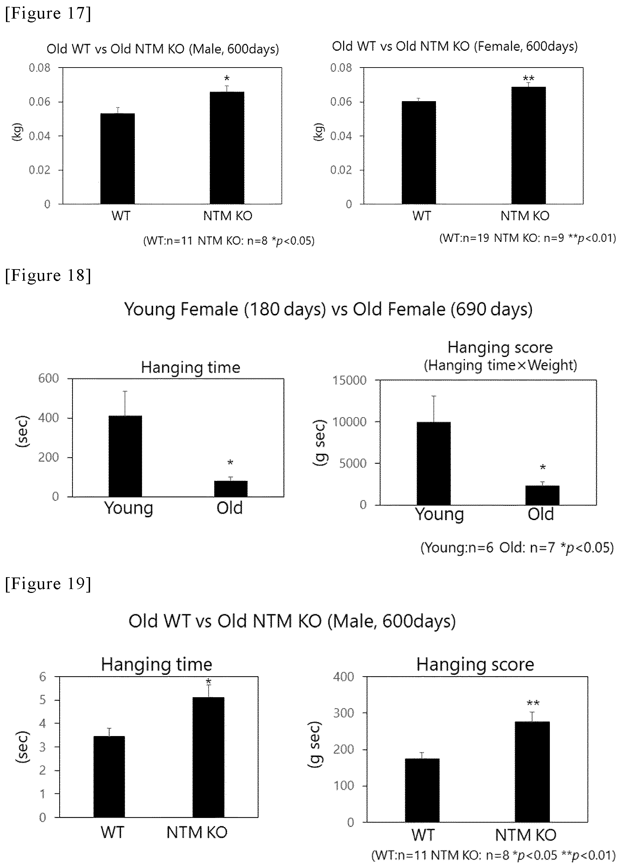

[Figure 17] Figure 17 shows the results obtained by investigating the influence of NTM deficiency on a reduction in muscle strength due to senescence. The left figure shows the results of comparing muscle strength between wild-type old male mice (WT) and NTM-deficient old male mice (NTM KO), whereas the right figure shows the results of comparing muscle strength between wild-type old female mice (WT) and NTM-deficient old female mice (NTM KO).
[Figure 18] Figure 18 shows the results obtained by comparing muscle endurance between young mice and old mice. The results obtained by comparing Hanging time (left figure) and the results obtained by comparing Hanging score (right figure) between wild-type young female mice (Young) and old female mice (Old) are shown.
[Figure 19] Figure 19 shows the results obtained by investigating the influence of NTM deficiency on a reduction in muscle endurance due to senescence (1). The results obtained by comparing Hanging time (left figure) and the results obtained by comparing Hanging score (right figure) between wild-type old male mice (WT) and NTM-deficient old male mice (NTM KO) are shown.

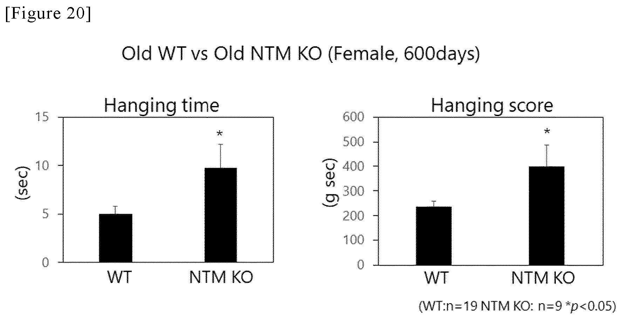

[Figure 20] Figure 20 shows the results obtained by investigating the influence of NTM deficiency on a reduction in muscle endurance due to senescence (2). The results obtained by comparing Hanging time (left figure) and the results obtained by comparing Hanging score (right figure) between wild-type old female mice (WT) and NTM-deficient old female mice (NTM KO) are shown.

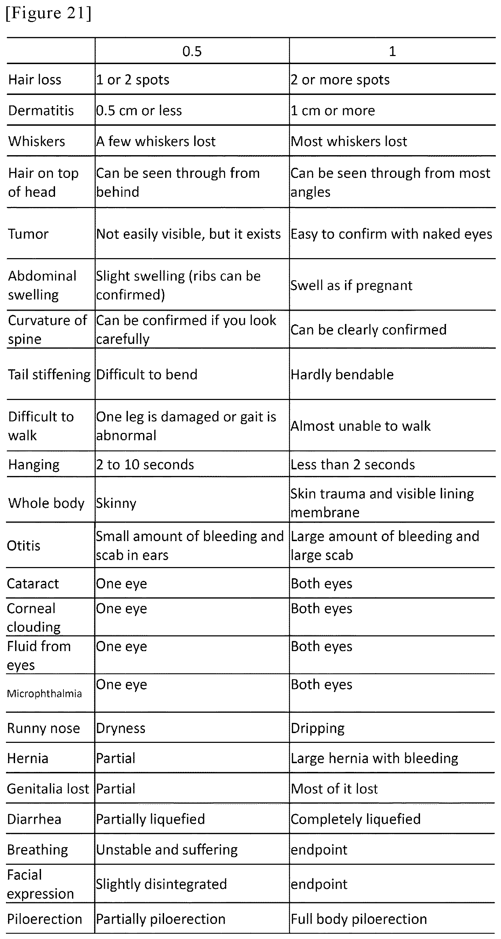

[Figure 21] Figure 21 shows the measurement items of Frailty score.

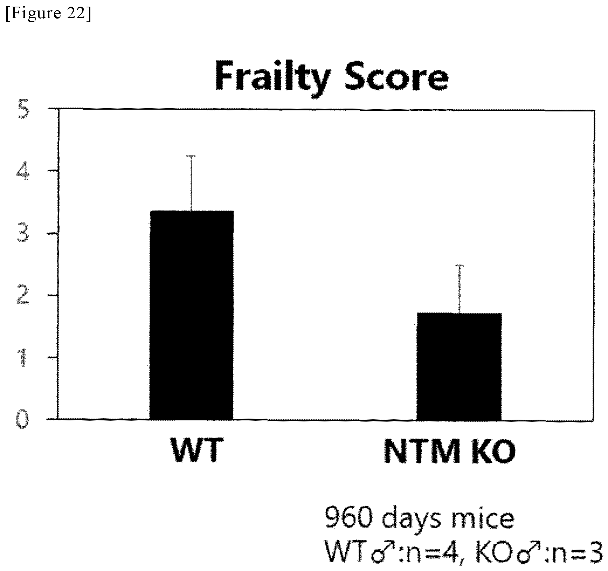

[Figure 22] Figure 22 shows the results obtained by investigating the influence of NTM deficiency on senescence-induced frailty syndrome. The results obtained by comparing frailty scores between wild-type old male mice (WT) and NTM-deficient old male mice (NTM KO).

### Description of Embodiments

Hereinafter, the embodiments of the present invention will be described.

A first embodiment relates to a cellular senescence-suppressing agent, comprising, as an active ingredient, an NTM (Neurotrimin) function inhibitor (hereinafter also referred to as "the cellular senescence-suppressing agent according to the present embodiment"). Herein, "cellular senescence suppression (suppression of cellular senescence)" means to prevent or to inhibit cells from undergoing senescence, or to improve cellular senescence.

Indicators of "cellular senescence" have been reported by many previous studies, and an indicator of cellular senescence has been the shortening of telomere length in the past, and other commonly used indicators may include an increase in the expression of p15 (CDKN2B) or p15 genes, an increase in the expression of p16 (CDKN2A) or p16 genes, an increase in the expression of p21 or p21 (CDKN1A) or p21 genes, an increase in the expression of p19 (CDKN2D) or p19 genes, activation of cellular senescence-specific β-galactosidase (SA-β-gal), disappearance of Lamin B1, formation of cellular senescence-specific heterochromatin (senescence-associated heterochromatic foci: SAHF), DNA damage response (DDR), and an increase in the expression or secretion of senescence-associated secretory phenotype (SASP)-related factors (e.g., IL-6, IL-8, TGF-β, etc.). As other indicators of cellular senescence, morphological changes such as increases in the size and granularity of cells, an increases in the size and number of mitochondria, and increased ROS production associated therewith, and also, activation of the retrotransposon LINE1, an increase in the histone acetyltransferase KAT7, etc. have been reported (for details regarding cellular senescence, see, for example, Gasek et al., Nature aging 1: 870-879, 2021, etc.).

In the present embodiment, the term "NTM" includes, for example, a human NTM protein (NCBI Accession Number: NM_001144058; NP_001137530), as well as homologs derived from other animals. Furthermore, NTM may be a protein having a sequence identity of 80% or more, and preferably a sequence identity of 90% or more (e.g., a sequence identity of 90% or more, 91% or more, 92% or more, 93% or more, 94% or more, 95% or more, 96% or more, 97% or more, 98% or more, or 99% or more) to the amino acid sequence of a human NTM protein, wherein the protein induces or promotes cellular senescence (i.e., a protein that causes a cell to undergo senescence (a cell exhibits characteristics of cellular senescence), when the protein is allowed to come into contact with the cell). It is to be noted that the secretory NTM protein is a protein obtained by removing a signal peptide region from the amino acid sequence shown in NCBI Accession No. NP_001137530, or a protein consisting of the amino acid sequence as set forth in SEQ ID No: 2. The NTM according to the present embodiment may also include a protein having a sequence identity of 80% or more, and preferably a sequence identity of 90% or more (e.g., a sequence identity of 90% or more, 91% or more, 92% or more, 93% or more, 94% or more, 95% or more, 96% or more, 97% or more, 98% or more, or 99% or more), to the amino acid sequence as set forth in SEQ ID No: 2, wherein the protein induces or promotes cellular senescence (i.e., a protein that causes a cell to undergo senescence (a cell exhibits characteristics of cellular senescence), when the protein is allowed to come into contact with the cell).

In the present embodiment, the "NTM function inhibitor" (hereinafter also referred to as "the NTM function inhibitor according to the present embodiment") means a substance that inhibits or suppresses the function (or activity) of NTM, particularly the function (or activity) of NTM to induce cellular senescence to a cell when the NTM acts on the cell. Examples of the substance that inhibits or suppresses NTM function may include, but are not particularly limited to: an antibody, a peptide aptamer, a specialty peptide (e.g., a peptide containing D-amino acid or N-methyl amino acid, etc., or a peptide having a macrocyclic skeleton, or the like; Cary et al., J. Synth. Org. Chem. Jpn., 75: 1171-1177, 2017), and a low-molecular-weight compound, which inhibit or suppress the function of NTM; a substance that decomposes NTM or induces decomposition of NTM; and a substance that inhibits or suppresses the expression of NTM (e.g., siRNA, miRNA, etc.). The "NTM function inhibitors" can be easily obtained by those skilled in the art by using the screening method according to a sixth embodiment.

A second embodiment relates to an antibody that suppresses or inhibits the function of NTM (hereinafter also referred to as "the anti-NTM antibody according to the present embodiment").

The "antibody" mentioned in the present description is not particularly limited in terms of the preparation method thereof and the structure thereof, and includes all "antibodies" that bind to desired antigens by desired properties, such as, for example, monoclonal antibodies, polyclonal antibodies, or nanoantibodies.

When the anti-NTM antibody according to the present embodiment is a polyclonal antibody, it can be prepared, for example, by injecting a mixture of an antigen and an adjuvant into an animal to be immunized (although it is not limited, but it is, for example, a rabbit, a goat, sheep, a chicken, a guinea pig, a mouse, a rat or a pig, etc.). Usually, an antigen and/or an adjuvant are injected into the subcutis or abdominal cavity of an animal to be immunized multiple times. Examples of the adjuvant may include, but are not limited to, a complete Freund's adjuvant and monophosphoryl lipid A synthetic-trehalose dicolinomicolate (MPL-TMD). After immunization with the antigen, an anti-NTM antibody can be purified from the serum derived from the immunized animal according to a usual method (for example, a method using Protein A-retaining Sepharose, etc.), or other methods.

On the other hand, when the anti-NTM antibody according to the present embodiment is a monoclonal antibody, it can be prepared, for example, as follows. Besides, the term "monoclonal" is used in the present description to suggest the properties of an antibody obtained from a substantially homogeneous population of antibodies (a population of antibodies, in which the amino acid sequences of the heavy and light chains constituting the antibodies are identical to one another), and thus, the term "monoclonal" should not be interpreted limitedly, such that the antibody is produced by a specific method (e.g., a hybridoma method, etc.).

Examples of the method of producing a monoclonal antibody may include a hybridoma method (Kohler and Milstein, Nature 256, 495-497, 1975) and a recombination method (U.S. Patent No. 4,816,567). Otherwise, the anti-NTM antibody according to the present embodiment may be isolated from a phage antibody library (e.g. Clackson et al., Nature, 352 624-628, 1991; Marks et al., J. Mol. Biol. 222, 581-597, 1991; etc.) or the like. More specifically, when a monoclonal antibody is prepared using a hybridoma method, the preparation method comprises, for example, the following 4 steps: (i) immunizing an animal to be immunized with an antigen; (ii) collecting monoclonal antibody-secreting (or potentially secreting) lymphocytes; (iii) fusing the lymphocytes with immortalized cells; and (iv) selecting cells that secrete a desired monoclonal antibody. As such an animal to be immunized, for example, a mouse, a rat, a guinea pig, a hamster, a rabbit or the like can be selected. After completion of the immunization, in order to establish hybridoma cells, lymphocytes obtained from the host animal are fused with an immortalized cell line, by using a fusion agent such as polyethylene glycol or an electrical fusion method. As fusion cells, a rat or mouse myeloma cell line is used, for example. After completion of the cell fusion, the cells are allowed to grow in a suitable medium that contains a substrate that inhibits the growth or survival of unfused lymphocytes and immortalized cell line. According to an ordinary technique, parent cells that lack the enzyme, hypoxanthine-guanine phosphoribosyl transferase (HGPRT or HPRT), are used. In this case, aminopterin is added to a medium that inhibits the growth of the HGPRT-deficient cells and allows the growth of hybridomas (HAT medium). From the thus obtained hybridomas, those producing desired antibodies can be selected, and then, a monoclonal antibody of interest can be obtained from a medium, in which the selected hybridomas grow, according to an ordinary method.

The thus prepared hybridomas are cultured *in vitro,* or are cultured *in vivo* in the ascites of a mouse, a rat, a guinea pig, a hamster, etc., so that an antibody of interest can be prepared from a culture supernatant or ascites.

The nanoantibody is a polypeptide consisting of the variable region of an antibody heavy chain (i.e. the variable domain of the heavy chain of a heavy chain antibody; VHH). In general, an antibody of a human or the like is composed of a heavy chain and a light chain. However, camelids such as llamas, alpacas and camels produce a single-chain antibody only consisting of a heavy chain (i.e., a heavy-chain antibody). Such a heavy-chain antibody can recognize a target antigen and can bind to the antigen, as in the case of an ordinary antibody consisting of a heavy chain and a light chain. The variable region of the heavy chain is a minimum unit having binding affinity for an antigen, and this variable domain fragment is referred to as a "nanoantibody." The nanoantibody has high heat resistance, digestion resistance and normal temperature resistance, and thus, it is possible to more easily prepare a large amount of the nanoantibody according to a genetic engineering method.

The nanoantibody can be produced, for example, as follows. A camelid is immunized with an antigen, and then, the presence or absence of an antibody of interest is detected in the collected serum. Thereafter, cDNA is produced from RNA derived from the peripheral blood lymphocytes of the immunized animal, in which a desired antibody titer is detected. A DNA fragment encoding VHH is amplified from the obtained cDNA, and is then inserted into a phagemid to prepare a VHH phagemid library. A desired nanoantibody can be produced from the prepared VHH phagemid library through several times of screening.

The anti-NTM antibody according to the present embodiment may be a genetically recombinant antibody. An example of such a genetically recombinant antibody may be, but is not limited to, a chimeric antibody consisting of a humanized antibody and a human antibody. The chimeric antibody means an antibody in which a variable region derived from an animal is ligated to a constant region derived from a different animal (for example, an antibody, in which a rat-derived antibody variable region is ligated to a human-derived antibody constant region) and the like (e.g. Morrison et al., Proc. Natl. Acad. Sci. U.S.A. 81, 6851-6855, 1984., etc.), and such a chimeric antibody can be easily constructed by a genetic recombination technique.

The humanized antibody is an antibody having a human-derived sequence in the framework region (FR) thereof and having a complementarity determining region (CDR) derived from another animal species (e.g., a mouse). Using herein a mouse as an example of another animal species, the humanized antibody can be produced by first transplanting the CDRs of the variable region of an antibody derived from a mouse into a human antibody variable region, then reconstituting the heavy chain and light chain variable regions, and then linking these humanized reconstituted human antibody variable regions to human antibody constant regions. The methods for producing such a humanized antibody is well known in the present technical field (e.g. Queen et al., Proc. Natl. Acad. Sci. USA, 86, 10029-10033, 1989., etc.).

The antigen-binding fragment of the anti-NTM antibody according to the present embodiment means a partial region of the anti-NTM antibody according to the present embodiment, which is an antibody fragment binding to NTM. Examples of such an antigen-binding fragment may include Fab, Fab', F(ab')₂, Fv (a variable fragment of an antibody), a single-chain antibody (a heavy chain, a light chain, a heavy chain variable region, a light chain variable region, a nanoantibody, etc.), scFv (single chain Fv), a diabody (an scFv dimer), dsFv (disulfide-stabilized Fv), and a peptide comprising the CDR of the anti-NTM antibody according to the present embodiment in at least a portion thereof.

Fab is an antibody fragment having antigen-binding activity obtained by digesting an antibody molecule with the protease papain, wherein about an N-terminal half of the heavy chain binds to the light chain as a whole via a disulfide bond. Fab can be produced by digesting an antibody molecule with papain to obtain a fragment thereof. Fab can also be produced by, for example, constituting a suitable expression vector into which DNA encoding the Fab has been inserted, then introducing the vector into suitable host cells (e.g. mammalian cells such as CHO cells, yeast cells, insect cells, etc.), and then allowing it to express in the cells.

F(ab')₂ is an antibody fragment having antigen-binding activity obtained by digesting an antibody molecule with the protease pepsin, which is slightly greater than Fab that binds to another Fab via a disulfide bond at hinge region. F(ab')₂ can be obtained by digesting an antibody molecule with the pepsin, or it can also be produced by binding Fab to another Fab via a thioether bond or a disulfide bond. Alternatively, F(ab')₂ can also be produced according to a genetic engineering method, as with Fab.

Fab' is an antibody fragment having antigen-binding activity obtained by cleaving the disulfide bond at hinge region of the above-described F(ab')₂. Fab' can also be produced according to a genetic engineering method, as in the case of Fab and the like.

scFv is an antibody fragment having antigen-binding activity that is a VH-linker-VL or VL-linker-VH polypeptide, wherein a single heavy chain variable region (VH) is ligated to a single light chain variable region (VL) using a suitable peptide linker. Such scFv can be produced by obtaining cDNAs encoding the heavy chain variable region and light chain variable region of an antibody and then treating them according to a genetic engineering method.

Diabody is an antibody fragment having divalent antigen-binding activity, in which scFv is dimerized. Regarding the divalent antigen-binding activity, this activity may be an activity of binding to either two identical antigens, or two different antigens. The diabody can be produced by obtaining cDNAs encoding the heavy chain variable region and light chain variable region of an antibody, then constructing cDNA encoding scFV, in which the heavy chain variable region is ligated to the light chain variable region using a peptide linker, and then treating the cDNA according to a genetic engineering method.

dsFv is a polypeptide which comprises a heavy chain variable region and a light chain variable region each including a substitution of one amino acid residue with a cysteine residue, in which VH binds to VL via a disulfide bond between the cysteine residues. The amino acid residue to be substituted with the cysteine residue can be selected based on prediction of the three-dimensional structure of antibodies. Such dsFv can be produced by obtaining cDNAs encoding the heavy chain variable region and light chain variable region of the antibody and then constructing DNA encoding the dsFv according to a genetic engineering method.

A peptide comprising CDR is constituted such that it comprises at least one region of CDR (CDR1-3) of the heavy chain or light chain. A peptide, which comprises multiple CDR regions, is able to bind to another peptide directly or via a suitable peptide linker. In the case of a peptide comprising CDR, DNA encoding the CDR of the heavy chain or light chain of the antibody is constructed, and it is then inserted into an expression vector. The type of the vector is not particularly limited, and it may be selected, as appropriate, depending on the type of a host cells into which the vector is to be introduced, and the like. The vector is introduced into host cells suitable for the expression of an antibody (e.g. mammalian cells such as CHO cells, yeast cells, insect cells, etc.), so that the peptide can be produced. Alternatively, such a peptide comprising CDR can also be produced by chemical synthesis methods such as an Fmoc method (fluorenylmethyloxycarbonyl method) and a tBoc method (t-butyloxycarbonyl method).

A human antibody (fully human antibody) generally has the same structure as the antibody of a human, in terms of the structures of a hypervariable region as an antigen-binding site in the V region), other parts in the V region, and a constant region. The human antibody can be easily produced by those skilled in the art using known techniques. The human antibody can be obtained, for example, by a method using a human antibody-producing mouse having a human chromosome fragment containing the H- and L-chain genes of a human antibody (e.g., Tomizuka et al., Proc. Natl. Acad. Sci. USA, 97, 722-727, 2000., etc.) or a method of obtaining a human antibody derived from a phage display selected from a human antibody library (see, for example, Siriwardena et al., Opthalmology, 109, 427-431, 2002., etc.).

The antigen-binding fragment of the anti-NTM antibody according to the present embodiment can be used to construct a multispecific antibody. Multispecificity means that an antibody has binding specificity to two or more antigens, and such multi specificity may be, for example, the form of a protein comprising a monoclonal antibody or an antigen-binding fragment, having binding specificity to two or more antigen. This multispecific antibody can be obtained by those skilled in the art using known techniques. Several methods have been developed as methods for constituting multi specificity, and such methods can be classified into: techniques of constructing asymmetric IgG, in which protein engineering operations are performed on two different types of antibody heavy chain molecules to allow the molecules to form a heterodimer; techniques of linking low-molecular-weight antigen-binding fragments obtained from an antibody to each other, or linking the low-molecular-weight antigen-binding fragment to another antibody molecule; and other techniques. As an example of a specific construction method, for example, the following publication can be referred to: Kontermann et al., Drug Discovery Today, 20, 838-847, 2015.

Examples of the anti-NTM antibody according to the present embodiment and an antigen-binding fragment thereof may include an anti-NTM antibody characterized in that the amino acid sequences of CDRs (complementarity determining regions) 1-3 satisfy any of the following (A), (B), (C), (D) or (E), and an antigen-binding fragment thereof:
(A) the antibody has:
   the amino acid sequence of heavy chain CDR1 being GYSFTSYW (SEQ ID No: 3),
   the amino acid sequence of heavy chain CDR2 being IYPGDSDTR (SEQ **ID** No: 4),
   the amino acid sequence of heavy chain CDR3 being ARQRFGEFAYYYYGLDV (SEQ ID No: 5),
   the amino acid sequence of light chain CDR1 being TGAVTTSNY (SEQ ID No: 6)
   the amino acid sequence of light chain CDR2 being GTS (SEQ ID No: 7), and
   the amino acid sequence of light chain CDR3 being ALWYSTHYV (SEQ ID No: 8);
(B) the antibody has:
   the amino acid sequence of heavy chain CDR1 being GYTLTELS (SEQ ID No: 9),
   the amino acid sequence of heavy chain CDR2 being FDPEDGETI (SEQ ID No: 10),
   the amino acid sequence of heavy chain CDR3 being ATYSSGWYALDY (SEQ ID No: 11),
   the amino acid sequence of light chain CDR1 being QSVSIY (SEQ ID No: 12)
   the amino acid sequence of light chain CDR2 being DAS (SEQ ID No: 13), and
   the amino acid sequence of light chain CDR3 being QQRIKWPFT (SEQ ID No: 14);
(C) the antibody has:
   the amino acid sequence of heavy chain CDR1 being GFTFSDYY (SEQ ID No: 15),
   the amino acid sequence of heavy chain CDR2 being ISNDGSTIY (SEQ ID No: 16),
   the amino acid sequence of heavy chain CDR3 being ARDYYGSGSFYPKPHY YYYYGMDV (SEQ ID No: 17),
   the amino acid sequence of light chain CDR1 being QSVLYSSNNKNY (SEQ ID No: 18),
   the amino acid sequence of light chain CDR2 being WAS (SEQ ID No: 19), and
   the amino acid sequence of light chain CDR3 being QQYYSPPFT (SEQ ID No: 20);
(D) the antibody has:
   the amino acid sequence of heavy chain CDR1 being GFSLSTSGVG (SEQ ID No: 21),
   the amino acid sequence of heavy chain CDR2 being IYWDDDKR (SEQ ID No: 22),
   the amino acid sequence of heavy chain CDR3 being AHSAYYDILTGYALDAFDI (SEQ ID No: 23),
   the amino acid sequence of light chain CDR1 being QSVSSY (SEQ ID No: 24) the amino acid sequence of light chain CDR2 being DAS (SEQ ID No: 25), and
   the amino acid sequence of light chain CDR3 being RQRSNWPWT (SEQ ID No: 26); or
(E) the antibody has:
   the amino acid sequence of heavy chain CDR1 being GFSLSTSGVG (SEQ ID No: 27),
   the amino acid sequence of heavy chain CDR2 being IYWDDDKR (SEQ ID No: 28),
   the amino acid sequence of heavy chain CDR3 being AHSAYYDILTGYALDAFDI (SEQ ID No: 29),
   the amino acid sequence of light chain CDR1 being QSVLYSSNNKIY (SEQ ID No: 30)
   the amino acid sequence of light chain CDR2 being WAS (SEQ ID No: 31), and the amino acid sequence of light chain CDR3 being QQYYSPPFT (SEQ ID No: 32).

Furthermore, the anti-NTM antibody according to the present embodiment may also include an antibody that competitively inhibits the binding between the antibody characterized in that it satisfies any of the above (A), (B), (C), (D) or (E), and NTM, and inhibits or suppresses the function of NTM (hereinafter also referred to as "the competitive antibody according to the present embodiment"). The competitive antibody according to the present embodiment can be prepared and obtained by performing competitive experiments, etc. that are well known to those skilled in the art. Specifically, when the binding between a first anti-NTM antibody (the anti-NTM antibody according to the present embodiment) and NTM is competitively inhibited by a second anti-NTM antibody, it is determined that the first anti-NTM antibody and the second anti-NTM antibody bind to antigen sites that are substantially identical to each other, or are extremely close to each other. Also, when the second anti-NTM antibody inhibits or suppresses the function of NTM, the second anti-NTM antibody is the competitive antibody according to the present embodiment and is included in the anti-NTM antibody according to the resent embodiment. Besides, as a method for the above-mentioned competitive experiment, for example, a method using a Fab fragment, etc. is generally applied in the present technical field. Please refer to, for example, WO95/11317, WO94/07922, WO2003/064473, WO2008/118356 and WO2004/046733, etc. Moreover, whether or not a concerned antibody inhibits or suppresses the function of NTM can be easily confirmed by the method disclosed in the Examples later, for example, by investigating whether or not the concerned antibody reduces the expression of a cellular senescence marker (p15, p16, p21, etc.)in cells, or whether or not the concerned antibody suppresses secretion of SASP-related factors from cells, or the like.

A third embodiment relates to a medicament or a composition (e.g., a pharmaceutical composition, a food and drink composition, a cosmetic composition, etc.), comprising the cellular senescence-suppressing agent according to the present embodiment (i.e., a cellular senescence-suppressing agent containing, as an active ingredient, an NTM function inhibitor) (hereinafter also referred to as "the medicament and the composition, etc. according to the present embodiment"). Otherwise, the third embodiment relates to a medicament or a composition, comprising an NTM function inhibitor as an active ingredient).

As described above, when cellular senescence is suppressed or inhibited, progression of individual aging is inhibited. In fact, the present inventors have confirmed that the decline in muscle strength and muscle endurance associated with aging is suppressed by inhibiting the function of NTM (see Examples). Moreover, the inventors have also confirmed that progression of frailty syndrome associated with aging can be suppressed by inhibiting the function of NTM (see the results of the experiment using knockout mice in Examples). Therefore, it is considered that progression of individual aging can be suppressed by using the cellular senescence-suppressing agent according to the present embodiment.

In addition, inhibition of NTM function is considered to be effective in treating diseases associated with aging (arteriosclerosis and osteoporosis, etc.). In tissues in which senescent cells exist, a chronic inflammatory state progresses, and this chronic inflammatory state is thought to be the cause of cancer and chronic inflammatory diseases. Furthermore, obesity is also considered to be a chronic inflammatory state. It has been reported that inflammation and cellular senescence are induced in biological tissues under an obese state, and that such an inflammatory state or cellular senescence, for example, cellular senescence in adipose tissues becomes a factor for acquisition of insulin resistance associated with obesity.

Accordingly, the medicament and the composition, etc. according to the third embodiment can be used as agents for improving individual aging or obesity, or as preventive agents or therapeutic agents for various types of disease provoked by cellular senescence, or as other agents. Various types of diseases provoked by cellular senescence are not particularly limited, and examples of the diseases may include various types of diseases developed due to aging, obesity and chronic inflammation, and metabolic disease. More specific examples of the diseases may include cancer, diabetes, hypertension, dyslipidemia, arteriosclerosis, cerebral infarction, heart disease (myocardial infarction, cardiomyopathy and cardiac hypertrophy), chronic obstructive pulmonary disease, chronic kidney disease, chronic liver disease (cirrhosis, hepatitis and fatty liver), dementia (Alzheimer's disease), Parkinson's disease, frailty syndrome, sarcopenia, emaciation, osteoarthritis, spondylosis deformans, skin disease, senile alopecia, cataracts, dry eyes, glaucoma, age-related macular degeneration, presbyopia, other chronic inflammatory diseases, and/or other metabolic diseases. Furthermore, according to recent reports, it has been elucidated that cellular senescence plays an important role in the severity of infectious diseases caused by the new coronavirus (SARS-CoV-2), and that the new coronavirus has the function of inducing cellular senescence (Lee et al., Nat. 599: 283-289, 2021). The medicament and the composition, etc. according to the third embodiment can also be used for the treatment of infectious diseases.

With regard to the medicament and the composition, etc. according to the present embodiment, the active ingredient itself (e.g., an NTM function inhibitor itself, or a cellular senescence-suppressing agent containing the NTM function inhibitor, or the like) may be administered. However, in general, the medicament and the composition, etc. according to the present embodiment may be desirably administered in the form of a composition comprising one or two or more pharmaceutical additives, as well as one or multiple substances serving as an active ingredient(s). The active ingredient(s) of the medicament and the composition, etc. according to the present embodiment may comprise a plurality of different cellular senescence-suppressing agents according to the present embodiment.

Examples of the dosage form of the medicament and the composition, etc. according to the present embodiment may include a tablet, a capsule, a granule, a powder agent, a syrup agent, a suspending agent, a suppository, an ointment, a cream agent, a gelling agent, a patch, an inhalant, an injection, and eye drops. These pharmaceutical preparations are prepared according to an ordinary method. In the case of a liquid agent, it may be a liquid agent in which the agent is dissolved or suspended in water or another suitable medium before use. Moreover, in the case of a tablet or a granule, it may be coated according to a well-known method. In the case of an injection, it is prepared by dissolving the active ingredient in water. The active ingredient may also be dissolved in a normal saline or a dextrose solution, as necessary, and a buffer or a preservative may also be added to such a solution.

The preparation for use in oral administration or parenteral administration is provided in any given pharmaceutical form. With regard to the pharmaceutical form of the preparation, the preparation can be prepared in the form of, for example, a pharmaceutical composition used for oral administration, such as a granule, a parvule, a powder medicine, a hard capsule, a soft capsule, syrup, an emulsion, a suspension or a liquid agent, or it can be prepared in the form of a pharmaceutical composition used for parenteral administration, such as an injection for intravenous administration, intramuscular administration or subcutaneous administration, a drop, a percutaneous absorption agent, a transmucosal absorption agent, a nasal agent, an inhalant, a suppository, eye drops, an ointment or a cream agent. In the case of an injection or a drop, it can be prepared in a powdery form such as a freeze-dried form, and it can be then dissolved in a suitable aqueous medium such as a normal saline before use.

The types of pharmaceutical additives used in the production of the medicament and the composition, etc. according to the present embodiment, the percentage of the pharmaceutical additives to the active ingredient, a production method thereof, and the like can be appropriately determined by those skilled in the art, depending on the form of the pharmaceutical composition to be produced. As such pharmaceutical additives, inorganic or organic substances, or solid or liquid substances can be used. In general, such pharmaceutical additives can be added at a weight percentage of 1% to 90% based on the weight of the active ingredient. Specific examples of such substances used as pharmaceutical additives include lactose, glucose, mannit, dextrin, cyclodextrin, starch, sucrose, magnesium aluminometasilicate, synthetic aluminum silicate, carboxymethylcellulose sodium, hydroxypropyl starch, carboxymethylcellulose calcium, ion-exchange resin, methyl cellulose, gelatin, gum Arabic, hydroxypropyl cellulose, hydroxypropylmethyl cellulose, polyvinylpyrrolidone, polyvinyl alcohol, light anhydrous silicic acid, magnesium stearate, talc, tragacanth, bentonite, Veegum, titanium oxide, sorbitan fatty acid ester, sodium lauryl sulfate, glycerin, fatty acid glycerin ester, purified lanolin, glycerinated gelatin, polysorbate, macrogol, vegetable oil, wax, liquid paraffin, white petrolatum, fluorocarbon, nonionic surfactant, propylene glycol, and water.

In order to produce a solid preparation used for oral administration, the active ingredient is mixed with an excipient ingredient such as lactose, starch, crystalline cellulose, calcium lactate or silicic acid anhydride to prepare a powder medicine. Otherwise, a binder such as saccharose, hydroxypropyl cellulose or polyvinylpyrrolidone, a disintegrator such as carboxymethyl cellulose or carboxymethylcellulose calcium, or other additives are further added to the above obtained mixture, as necessary, and the obtained mixture is then subjected to wet granulation or dry granulation, so as to prepare a granule. In order to produce a tablet, such a powder medicine or a granule may also be subjected to a tablet-making operation, directly or with addition of a lubricant such as magnesium stearate or talc. The prepared granule or tablet may be coated with an enteric coating base such as hydroxypropylmethylcellulose phthalate or a methacrylic acid-methyl methacrylate polymer, so as to prepare an enteric coated drug. Otherwise, it may be coated with ethyl cellulose, carnauba wax or hydrogenated oil, so as to prepare a long-acting preparation. Moreover, in order to produce a capsule, a powder medicine or a granule is filled into a hard capsule, or the active ingredient is coated with a gelatin film, directly or after being dissolved in glycerin, polyethylene glycol, sesame oil, olive oil or the like, thereby producing a soft capsule.

Among preparations for use in parenteral administration, in order to produce an injection, the active ingredient, together with a pH adjuster such as hydrochloric acid, sodium hydroxide, lactose, lactic acid, sodium, sodium monohydrogen phosphate or sodium dihydrogen phosphate, and an isotonizing agent such as sodium chloride or glucose, as necessary, is dissolved in a distilled water for injection, and the obtained solution is subjected to aseptic filtration and is then filled into an ampule. Otherwise, mannitol, dextrin, cyclodextrin, gelatin or the like may be further added to the above obtained solution, and the obtained mixture may be then subjected to vacuum freeze-drying, so as to prepare an injection which is dissolved before use. Furthermore, lecithin, polysorbate 80, polyoxyethylene hydrogenated castor oil or the like is added to the active ingredient, and it is emulsified in water, so as to prepare an emulsion for injection.

Eye drops may be produced in the form of a liquid in which the active ingredient is suspended or dissolved in an aqueous solvent (e.g., a phosphate-buffered saline).

An ointment and a cream agent can be produced by kneading and mixing the active ingredient with a base agent and additives. An oil-based ointment can be produced, for example, by heating and melting an oil-based base agent such as oils and fats, waxes, or hydrocarbons such as paraffin, adding the active ingredient to the oil-based base agent, blending the mixture for dissolution or dispersion, and mixing and kneading the thus obtained mixture until the whole becomes homogeneous. A water-soluble ointment can be produced, for example, by heating and melting a water-soluble base agent such as macrogol, adding the active ingredient thereto, and mixing and kneading the obtained mixture until the whole becomes homogeneous.

A cream agent can be produced, for example, by adding the active ingredient to an oil phase consisting of Vaseline or higher alcohol, or an oil phase comprising it with additives such as an emulsifier, or to an aqueous phase consisting of purified water , or an aqueous phase comprising it with additives such as an emulsifier, then heating the mixture, and then combining the oil phase and the water phase and emulsifying them by stirring until the entire mixture becomes homogeneous.

In order to produce a parenteral rectal administration agent, the active ingredient, together with a suppository base agent such as cacao butter, tri-, di- and mono-glyceride of fatty acid, or polyethylene glycol, is moisturized to be dissolved, and the resultant is then poured into a mold, followed by cooling. Otherwise, the active ingredient may be dissolved in polyethylene glycol or soybean oil or the like, and it may be then coated with a gelatin film or the like.

The applied dose and the number of doses of the medicament and the composition, etc. according to the present embodiment are not particularly limited. The applied dose and the number of doses can be appropriately selected, depending on the purpose of preventing and/or treating the conditions to be improved, or deterioration and/or progression of the disease to be treated, the type of the disease, and the body weight and age of a patient, according to the judgment of a doctor.

Generally, the dose applied for an adult per day by oral administration is approximately 0.01 to 1000 mg (the weight of the active ingredient), and this dose can be administered once or divided over several administrations per day, or every several days. In the case of using the medicament or the pharmaceutical composition as an injection, the injection is desirably administered to an adult continuously or intermittently at a daily dose of 0.001 to 100 mg (the weight of the active ingredient).

The medicament and the composition, etc. according to the present embodiment may be prepared as a sustained release formulation, such as an implant tablet and a delivery system encapsulated into a microcapsule, by using a carrier capable of preventing the prompt removal of the agent from the body. As such carriers, biodegradable, biocompatible polymers, such as ethylene vinyl acetate, polyanhydride, polyglycolic acid, collagen, polyorthoester and polylactic acid, can be used. These materials can be easily prepared by a person skilled in the art. Moreover, a liposome suspension can also be used as a pharmaceutically acceptable carrier. Liposome is prepared as a lipid composition comprising, but are not limited thereto, phosphatidylcholine, cholesterol and PEG-derived phosphatidylethanol (PEG-PE), by being passed through a filter with a suitable pore size, so that it can have a size suitable for the use thereof, and it is then purified by a reverse phase evaporation method.

The medicament and the composition, etc. according to the present embodiment may be provided in the form of a kit, together with an instruction manual regarding an administration method and the like. The agent included in the kit is supplied with a vessel, in which the activity of the components of the therapeutic agent, etc. is effectively sustained for a long period of time, the agent and the like are not adsorbed on the inside thereof, and the vessel is produced from materials that do not degrade the components. For example, a sealed glass ampule may comprise a buffer or the like that has been enclosed in the presence of neutral and unreactive gas such as nitrogen gas. Moreover, the instruction manual included with the kit may be printed on a paper or the like, or may also be stored in an electromagnetically readable medium such as CD-ROM or DVD-ROM, and may be then supplied to users. Alternatively, the instruction manual may also be an electronic file available via the Internet or the like.

When the composition according to the present embodiment is provided as a food and drink composition, the form thereof is not particularly limited. Examples of the form of such a food and drink composition may include beverages such as soft drinks and nutritional drinks, confectioneries such as candy, gum, jelly, cream and ice cream, dairy products such as milk drinks, fermented milk, drinking yogurt and butter, and other supplements (nutritional supplements). The food and drink composition according to the present embodiment is not particularly limited, and examples of the present food and drink composition may include anti-aging supplements for the purpose of preventing or inhibiting individual aging, diet supplements (body weight reduction), supplements for lowering blood sugar levels, and supplements for maintaining or increasing muscle strength.

A fourth embodiment relates to a method of suppressing or inhibiting cellular senescence *in vivo* or *in vitro,* comprising suppressing or inhibiting the function (or activity) of NTM. Herein, as described above, the function (or activity) of NTM means to the function (or activity) of acting on a cell to induce cellular senescence in the cell. For example, when cellular senescence is suppressed in a living body, an NTM inhibitor (for example, the antibody according to the second embodiment, or a low molecular weight compound that inhibits the function of NTM, etc.) may be administered to the living body together with a pharmaceutically acceptable carrier and the like.

Also, the fourth embodiment includes the use of an NTM inhibitor to suppress or inhibit cellular senescence *in vitro* or *in vivo.*

It is to be noted that, in the fourth embodiment, when cellular senescence is suppressed *in vivo,* medical procedures are excluded from the acts. Also, when an NTM inhibitor is used *in vivo,* the use thereof as a medical procedure is excluded. Accordingly, the fourth embodiment may include a step of administering a food and drink composition or a cosmetic composition, etc. comprising an NTM inhibitor to a living body (e.g. oral administration (for example, taking a supplement) or transdermal administration (for example, application of a cosmetic composition onto the ski, etc.) or the like) for anti-aging with the purpose of preventing or preventing individual aging.

However, needless to say, the preventive method and the therapeutic method according to a fifth embodiment include the use of an NTM inhibitor as a medical procedure.

A fifth embodiment relates to a method of improving individual aging, a method of improving obesity, or a method of preventing or treating a disease provoked by cellular senescence, each of which comprises administering the medicament, etc. according to the present embodiment (i.e., a medicament or a pharmaceutical composition containing the cellular senescence-suppressing agent according to the present embodiment) to a subject (a subject affected with a disease to be prevented or treated).

Herein, "improvement" means making the physical condition at a certain point in time more favorable, and the improvement of individual aging may include, for example, preventing decline in physical ability associated with aging or slowing the speed of the decline in physical ability, and the improvement of obesity may include, for example, body weight reduction as well as the improvement of metabolic syndrome, but is not limited to these concepts.

"Treatment" means preventing or alleviating the progression and deterioration of a pathological condition caused in a mammal as a therapeutic subject. "Prevention" means preventing, in advance, the onset of a disease in a mammal at risk of developing the disease.

The subject, to which the medicament, etc. of the present embodiment is administered, may be any animal classified as a mammal, and is not particularly limited. Examples of the subject may include humans, as well as animals belonging to primates, including pet animals such as dogs, cats, rabbits and ferrets, and livestock animals such as cows, pigs, sheep and horses. Humans are particularly preferable as subjects to which the medicament, etc. of the present embodiment is administered.

A sixth embodiment relates to a method relates to a method of screening for a substance that suppresses cellular senescence, comprising searching for an NTM function inhibitor. More specifically, the sixth embodiment relates to a method of screening for a substance that suppresses cellular senescence, comprising the following (a) and (b):
(a) a step of allowing NTM and a candidate substance to come into contact with cells; and
(b) a step of evaluating the degree of cellular senescence of the cells with which the NTM and the candidate substance are allowed to come into contact.

It is considered that NTM acts on other cells or on cells themselves expressing NTM, and induces senescence to those cells. Therefore, the substance that inhibit the function of NTM is considered to suppress cellular senescence. In fact, it has been revealed by the present invention that an antibody against NTM (a neutralizing antibody) suppress the expression of cellular senescence markers (for example, p15, etc.) in senescence-induced cells, and suppresses secretion of SASP-related factors (for example, IL-6, etc.) from the cells (see the Examples for details).

The step (a) is a step of allowing NTM and a candidate substance that suppresses cellular senescence to come into contact with cells in which cellular senescence has been induced. Specifically, for example, the step (a) can be performed by adding NTM (e.g., secretory NTM) and a candidate substance in an appropriate amount to a medium in which cells are cultured, and culturing the cells at a suitable temperature. In some cases, cells in which cellular senescence has already been induced may be used. In this case, induction of cellular senescence can be carried out, for example, by allowing a Ras protein to express in the cells, without any particular limitation.

With regard to the order in which NTM and a candidate substance that suppresses cellular senescence are allowed to come into contact with cells, either one may come first, or they may be allowed to simultaneously come into contact with the cells. Otherwise, the NTM and the candidate substance may be mixed in advance, and then, the mixture may be allowed to come into contact with the cells.

The step (b) is a step of examining whether the candidate substance suppresses the induction of cellular senescence by NTM. Herein, whether the cells have undergone senescence can be evaluated using, as indicators, the shortening of telomere length, increased expression or activation of cellular senescence markers such as p15, p16, p21, p19 and SA-β-gal, the disappearance of Lamin B1, and increased secretion of SAHF, DDR, or such SASP-related factors as SIL-6, IL-8 and TGF-β, etc., as mentioned above. Using these indicators, when a candidate substance is allowed to come into contact with cells and as a result, the characteristic amount of cellular senescence in the cells contacted with the candidate substance is decreased compared to cells not contacted with the candidate substance, it can be determined that the candidate substance suppresses cellular senescence or is likely to suppress cellular senescence.

When the present description is translated into English and the English description includes singular terms with the articles "a," "an," and "the," these terms include not only single items but also multiple items, unless otherwise clearly specified from the context that it is not the case. In addition, in the present description, the term "about" is used to mean a numerical range of ±10%.

Hereinafter, the present invention will be further described in the following examples. However, these examples are only illustrative examples of the embodiments of the present invention, and thus, are not intended to limit the scope of the present invention.

### Examples

### 1. Materials and Methods

### 1-1. Cell culture reagents

Powdered MEM (Minimum essential medium), powdered DMEM (Dulbecco's modified eagle's medium), and powdered RPMI 1640 (Roswell Park Memorial Institute 1640) medium were purchased from Nissui Pharmaceutical Co., Ltd. Penicillin and streptomycin (P/St) were purchased from Meiji Seika Kaisha, Ltd. Fetal bovine serum (FBS) was purchased from Equitech-Bio or GIBCO. Trypsin powders were purchased from GIBCO. RNAi library, siRNA against human Neurotrimin (NTM), and control siRNA thereof were purchased from Thermo Fisher Scientific. As other experimental reagents that were not particularly specified, special-grade or biochemical experimental-grade reagents purchased from Wako, Nacalai Tesque, or SIGMA were used.

### 1-2. Preparation of cell culture reagents

### PBS(-) Solution

NaCl, KCl, Na₂HPO₄, and KH₂PO₄ were dissolved in MilliQ water to concentrations of 137 mM NaCl, 2.7 mM KCl, 10 mM Na₂HPO₄, 1.76 mM and KH₂PO₄, and the obtained solution was autoclaved, and was then preserved at room temperature.

### 200 mM L-Glutamine

L-glutamine was dissolved in MilliQ to make 200 mM, filter sterilized, and stored at 4°C.

### 10% NaHCO₃

NaHCO₃ was dissolved in MilliQ water, and the obtained solution was autoclaved, and was then preserved at 4°C.

### DMEM (High Glucose)

4.75 g of powdered DMEM was dissolved in 500 mL of MilliQ water, and the obtained solution was stirred for 30 minutes, and was then autoclaved. Thereafter, 10 mL of 200 mM L-Glutamine and 8.75 mL of 20% Glucose were added thereto. Further, a 10% NaHCO₃ solution was added in an appropriate amount to the mixed solution, until the medium turned red. The thus obtained medium was used as DMEM (High Glucose).

### MEM

4.7 g of powdered MEM was dissolved in 500 mL of MilliQ water, and the obtained solution was stirred for 30 minutes, and was then autoclaved. Thereafter, 5 mL of 200 mM L-Glutamine was added thereto. Further, a 10 % NaHCO₃ was added in an appropriate amount to the mixed solution, until the medium turned red. The thus obtained medium was used as MEM.

### RPMI 1640

5.1 g of powdered RPMI 1640 was dissolved in 500 mL of MilliQ water, and the obtained solution was stirred for 30 minutes, and was then autoclaved. Thereafter, 5 mL of 200 mM L-Glutamine was added thereto. Further, a 10% NaHCO₃ solution was added in an appropriate amount to the mixed solution, until the medium turned red. The thus obtained medium was used as RPMI 1640.

### Fetal bovine serum (FBS)

A vessel containing serum frozen at -20°C was thawed in a 37°C hot water bath, and the serum was then incubated at 56°C for 30 minutes to inactivate complement components.

### Penicillin-streptomycin solution (P/St)

The medium was dispensed in an amount of 500 µL each, and was then preserved at -20°C. When used, penicillin and streptomycin was added to the medium in an amount of 1/1000 (final concentration: 100 units/mL penicillin, 100 µg /mL streptomycin).

### Polybrene (hexadimethrine bromide)

Polybrene was adjusted to 10 mg/mL with MilliQ water, and was then sterilized by filtration through a 0.20 µm filter (prepared just before use).

### 4-OH Tamoxifen

4-OH Tamoxifen was adjusted to 1 mM with ethanol, and was then preserved at -20°C. When used, the obtained solution was added to the medium in an amount of 1/5000 (final concentration: 200 nM).

### 1-3. Plasmid

pCMV-Flag-NTM, pBabe-Flag-NTM (Puro), and pET38a-His-NTM were prepared by ordinary methods, were stored, and were then used. pLNCX2 ER:Ras was purchased from Addgene as a plasmid for expressing a Ras protein.

### 1-4. Preparation of recombinant NTM protein

The plasmid carrying secretory NTM was prepared by incorporating the cDNA sequence of secretory NTM (SEQ ID No: 1) into a pET38a vector and adding a His tag to the N-terminus. the *Escherichia coli* strain BL21 was transformed with the prepared plasmid, and the resulting transformant was cultured in a large amount. A secretory NTM protein of interest was concentrated and purified from a cell mass after culture, using His beads. The obtained protein was dialyzed overnight against PBS(-) using a nitrocellulose membrane, and the concentration of the protein was measured by a BCA method.

### 1-5. Preparation of anti-NTM human monoclonal antibody

Preparation of an anti-NTM human monoclonal antibody was outsourced to Trans Chromosomics, Inc., and was carried out using the fully human antibody-producing mice TC-mABTM. As an antigen, there was used the CHO cells in which full-length NTM was forcibly expressed or a secretory recombinant NTM protein purified, using His beads, from a culture supernatant of Expi293F cells in which NTM was forcibly expressed. The fact that the prepared antibody recognizes the NTM protein was confirmed by using HeLa cells or CHO cells, in which NTM was forcibly expressed, and using, as an indicator, the staining of the cell membrane of each cell in a live cell state.

### 1-6. Analysis using cells

### 1-6-1. Cell culture

### IMR90 cells

IMR90 cells were cultured at 37°C in a low-oxygen incubator of 5% CO₂ and 3% O₂, using MEM, 10% FBS, 1 x MEM Non-Essential Amino Acids Solution, and 1 mM Sodium Pyruvate, and thereafter, the cells were passaged at a ratio of 1 x 10⁶ cells/dish.

### HeLa cells

HeLa cells were cultured at 37°C in an incubator of 5% CO₂, using MEM and 10% FBS, and thereafter, the cells were passaged at a ratio of 1 x 10⁶ cells/dish.

### Plat A cells

Plat A cells were cultured at 37°C in an incubator of 5% CO₂, using DMEM (High Glucose) and 10% FBS, and thereafter, the cells were passaged at a ratio of 1 x 10⁶ cells/dish.

### MEFs

MEFs were cultured at 37°C in an incubator of 5% CO₂, using DMEM (High Glucose), 10% FBS, and P/St, and thereafter, the cells were passaged at a ratio of 1 x 10⁶ cells/dish.

### Hybridomas

Hybridomas produced by Trans Chromosomics were cultured at 37°C in an incubator of 5% CO₂, using PRMI1630, 10% FBS, P/St, HT medium supplement Hybri-MaxTM, and 5% Briclone.

### 1-6-2. Real-time PCR

The mRNA expression level of each gene was quantified using SYBR Green (Roche). The basic composition of the reaction solution is shown below.
Power SYBR Green PCR Master Mix: 5 µL
5 µM Forward primer: 1 µL
5 µM Reverse primer: 1 µL
Template cDNA:3 µL

The above-described reaction solution was blended in a Light Cycler 480 Multiple plate 96, white (Roche), was then spun down, and was then reacted and quantified using a Light Cycler 480 (Roche). A ΔCT method was used for quantification. The amount of the mRNA of each gene was corrected by dividing by the amount of the mRNA of HPRT1 or s17. Triplicate samples were used for a single trial, and the mean value and the standard deviation were calculated. Student's t-test was used to the test for significant difference.

### 1-6-3. Gene transfer using PEI (polyethylenimine)

Gene transfer using PEI was carried out in the case of using Plat A cells. Hereafter, a gene transfer method using a 10 cm dish is described.

20 µg of plasmid DNA was added to 1000 µL of Opti-MEM (GIBCO), and was gently mixed to prepare DNA Mix. On the other hand, 60 µL of PEI was added to 1000 µL of Opti-MEM, and was gently mixed to prepare PEI Mix. After 5 minutes, the DNA Mix and the PEI Mix were mixed with each other, and the obtained mixture was then incubated at room temperature for 20 minutes. 500 µL of the resulting DNA complex solution was added to cells cultured in 10 mL of a medium, so that it was evenly distributed. The cells were cultured at 37°C in a 5% CO₂ incubator, and after 12 hours, the medium was replaced with a basal medium.

### 1-6-4. Gene transfer using retroviral vectors

On the day before transfection, Plat A cells, which are virus packaging cells derived from HEK293-T cells, were seeded on a 100 mm dish to a density of 2 x 10⁶ cells/dish. On the following day, 30 µg each of retroviral vector was transferred using PEI. Hereafter, the operations from gene transfer to infection into IMR90 cells were performed at the P2 level.

Twelve hours after the gene transfer, the medium was replaced with MEM medium (10% FBS, 1 x NEAA, and 1 mM sodium pyruvate) (10 mL/dish), and further 24 to 36 hours later, the culture supernatant was collected. The obtained culture supernatant was sterilized by filtration through a 0.45 µm filter (Advantec), and was then used as a retrovirus stock solution (preserved at -80°C). A 10 mg/mL polybrene solution that had been prepared just before use was added to the obtained retrovirus solution to a final concentration of 10 µg/mL, so as to prepare a retrovirus solution.

On the day before infection, IMR90 cells were seeded in a 6-well plate to a density of 2 x 10⁵ cells/well. On the following day, the retrovirus solution was added in an amount of 2 mL/well to the plate, and the obtained mixture was then centrifuged at 1,370 x g (at 3,500 rpm with PlateSpin II (Kubota)) at room temperature for 90 minutes. After the reaction mixture had been cultured for 8 hours at 37°C in a 5% CO₂ incubator, the medium was replaced with a normal medium for IMR90 cells.

Two days after the infection, the infected cells were selected by culturing them, while performing two passaging operations, in a medium containing neomycin or puromycin.

### 1-6-5. Knockdown method using Lipofectamin RNAi MAX

Knockdown of specific genes using Lipofectamin RNAi MAX (Invitrogen) was performed using IMR90 cells. Hereafter, an RNAi introduction method in the case of using a 12-well plate is described.

10 pmol of siRNA (Thermo Fisher) was added to 100 µL of Opti-MEM (GIBCO), and the obtained mixture was gently blended to prepare RNA Mix. On the other hand, 3 µL of Lipofectamin RNAi MAX was added to 100 µL of Opti-MEM, and the obtained mixture was gently blended to prepare Lipofectamin Mix. After 5 minutes, the RNA Mix and the Lipofectamin Mix were mixed with each other, and the obtained mixture was then incubated at room temperature for 20 minutes. 200 µL of the resulting RNA complex solution was added to cells cultured in 2 mL of a medium from which P/St was excluded, so that it was evenly distributed. The cells were cultured at 37°C in a 5% CO₂ incubator, and after 12 hours, the medium was replaced with a basal medium.

### 1-6-6. Cellular senescence induction experiment using Ras-induced IMR90 cells

pLNCX2 ER:Ras was introduced into Plat A cells, so that the cells were allowed to generate retrovirus. After IMR90 cells had been infected with the generated retrovirus, the infected cells were selected in the presence of neomycin, and the obtained cells were used as IMR90 ER-Ras.

Cellular senescence associated with Ras induction was induced by allowing 200 nM 4-OH tamoxifen to act on the IMR90 ER-Ras cells for 4 to 6 days.

### 1-7. Analysis using mice

### 1-7-1. Rearing of wild-type and NTM-deficient mice

The frozen embryos of NTM-deficient mice were obtained from the Mutant Mouse Resource and Research Center (MMRRC). NTM-deficient mice were reared and bred after sufficient backcrossing with C57B6 mice had been carried out. The mice were reared under SPF conditions under a 12-hour light/dark cycle lighting, and were allowed to freely consume solid food CE-2 (CLEA) and water (autoclaved distilled water). Soft tips sterilized by dry heat sterilization were used for the bedding, and cages and drinking bottles were purchased from Natsume Seisakusho Co., Ltd. and CLEA Japan, Inc., respectively. All experiments were performed using littermates obtained by mating male and female NTM hetero-deficient mice. All animal experiments were performed in accordance with the University of Tokyo Animal Experiment Implementation Regulations.

### 1-7-2. Genotyping

An ear punch method was used for individual identification. Approximately 2 mm of the tail was cut from approximately 4-week-old mice, using surgical scissors, and it was collected in a 1.5 mL tube. 600 µL of 50 mM NaOH was added thereto, and the mixture was then heated in a heat block at 90°C for 30 minutes. After the heat treatment, the reaction mixture was vortexed for 10 seconds, and the solution was thoroughly stirred. Thereafter, 50 µL of 1 M Tris-HCI (pH 8.0) was added to the reaction mixture, and the thus obtained mixture was fully vortexed again. After that, the reaction mixture was centrifuged at 15,000 rpm at 4°C for 10 minutes. The obtained supernatant was used as a template genome DNA solution in the following PCR reaction. The primers that specifically recognize individual alleles of the wild type and NTM KO are shown below.
[NTM (wild-type allele)]
   Sense: 5'- CTGAACTGCTACGGGGAAGAG -3' (SEQ ID No: 33)
[Knockout allele]
   Sense: 5'- GCAGCGCATCGCCTTCTATC -3' (SEQ ID No: 34)
[Common allele (Reverse)]
   Antisense: 5'- CTCGGGAAAAGGCAGTAGCAG -3' (SEQ ID No: 35)

### 1-7-3. Preparation of mouse embryonic fibroblasts (MEFs)

A trypsin solution was prepared by dissolving trypsin powders (GIBCO) in PBS (-) to a concentration of 0.25% (w/v), and then sterilizing the obtained solution by filtration through a 0.45 µm filter (Advantec) (prepared just before use).

13.5 Days after mating of female and male NTM hetero-deficient mice, the whole body of the female NTM hetero-deficient mice was disinfected with 70% ethanol, and the abdomen was opened to remove the uterus containing fetuses. After cutting the base of the umbilical cord with scissors, the fetuses were removed one by one from the uterus, and were then immersed in PBS (-). After removing the head, internal organs, limbs, and tail from each fetus, they were transferred into 1 mL of an ice-cold trypsin solution, and were then minced to 2 to 3 mm pieces with sharp scissors. The fetal pieces were transferred into a 15 mL tube, the liquid amount per fetus was adjusted to 5mL with a trypsin solution, and the solution was then shaken at 37°C at 60 to 100 cycles/min. for 10 minutes, while checking the degree of digestion.
Thereafter, in order to terminate the trypsin reaction, 1 mL of FBS was added to the solution, and the cells were thoroughly suspended. Further, in order to remove cell masses, the solution was filtered through a 100 µm cell strainer (Falcon) mesh. The filtered cell suspension was centrifuged (280 x g, 5 minutes, 4°C), the supernatant was removed, and the resulting precipitate was suspended in a basal medium (DMEM (High Glucose), 10% FBS, and P/St) and was seeded on a 100 mm dish at a ratio of one well per fetus. The medium was replaced on the next day, and the cells were seeded again by a trypsin treatment and was then used in the experiment.

### 1-7-4. Preparation of mouse serum and measurement of serum components

Mouse blood was collected from the inferior vena cava using a syringe and a 23G needle under anesthesia. After the blood collection, the blood was transferred into a 1.5 mL tube, was stirred by inversion, and was then left at rest at normal temperature for 1 hour. After leaving it at rest, the blood was centrifuged at 4°C at 3000 rpm for 30 minutes, and the supernatant fraction was collected to obtain serum. For the measurement of hemoglobin A1c, a mixture obtained by mixing 30 µL of the mouse blood with 5 µL of 10 mM EDTA was used. The measurement of mouse blood components was outsourced to Nagahama Life Science Laboratory. Student's t-test was used to the test for significant difference.

### 1-7-5. Insulin tolerance test (ITT)

Wild-type and NTM-deficient mice were fasted for 4 hours under single-housed conditions, and then, Human recombinant insulin (Invitrogen) diluted with PBS was intraperitoneally administered to the mice to achieve 1 IU (International Unit)/kg. Thereafter, blood was collected from the tail vein over time, at 0, 30, 60, 90, and 120 minutes, and blood glucose levels were measured using an Ascensia Breeze 2 (Bayer Yakuhin, Ltd.). Student's t-test was used to the test for significant difference.

### 1-7-6. Rearing and dissection of db/db mice

db/db mice (BKS Cg. db/db) used as obesity mouse models were purchased from CLEA Japan, Inc. As a control group, control mice (BKS Cg. m+/m+) of the db/db mice were also purchased from CLEA Japan, Inc. and were used in the experiment. The two types of mice were reared under single breeding conditions until 15 weeks of age, were then fasted for 6 hours, and were then sacrificed, and thereafter, individual organs were collected.

### 1-7-7. Consumed calorie restriction experiment in mice

C57BL6J male mice purchased from CLEA Japan, Inc. were divided into two groups so that the body weights became equal between these groups, and the mice were then reared alone. A free-feeding group was allowed to eat freely under conditions of a sufficient amount of food provided, and the weekly food intake was recorded. On the other hand, in a consumed calorie restriction group, the mice were fed three times a week, so that the food consumed by the calorie restriction group became 60% of the weight of the food consumed by the free-feeding group. Both groups were weighed over time and were reared for 210 days. The mice were fasted for 2 hours before sacrifice, and thereafter, their organs were collected.

### 1-7-8. High-fat diet loading experiment in mice

8 to12 Male wild-type mice and NTM-deficient mice aged 4 to 6 weeks were reared alone and were fed with a high-fat diet (60 kcal % Fat, High Fat Diet: HFD) (Research diets), and their body weight and food intake were measured for 9 weeks. After 9 weeks of the high-fat diet feeding, the mice were fasted for 6 hours and were then sacrificed.

### 1-7-9. Measurement of muscle strength in old mice

The muscle strength (grip strength) of 600-day-old wild-type and NTM-deficient old mice was measured by a Grip Strength Test. The Grip Strength Test was performed using a smart rat/mouse grip strength measuring device MK-380Si (Muromachi Kikai Co., Ltd.). Three measurements were performed for a single mouse, and the mean value was used as a grip strength value for each mouse. Student's t-test was used to test for significant difference.

### 1-7-10. Measurement of muscle endurance in old mice

The muscle endurance of 600-day-old wild-type and NTM-deficient old mice was measured by a 4-limb Hanging Test (Hanging test). The test was performed using a wire hanging experimental box (O'HARA & CO., LTD.), and the wire mesh was turned upside down together with a mouse under the condition where the mouse was holding the wire mesh with all of the limbs, and the time at which the mouse could endure before it fell was defined to be the endurance time (Hanging time). Furthermore, a score obtained by multiplying this endurance time by the body weight of each mouse was evaluated as a hanging score. Student's t-test was used to test for significant difference.

### 1-7-11. Calculation of frailty score

The frailty scores of 960-day-old wild-type and NTM-deficient male mice were calculated. The indicator of frailty score used was a partially revised version of a previous paper (Whitehead et al., J Gerontol A Biol Sci Med Sci. 69, 621-32, 2014). Each item was evaluated on a three-point scale of 0, 0.5, and 1 for a single mouse, and a total value of all 23 items was calculated as frailty score for each mouse. The measurement items of frailty score are shown in Figure 21.

### 2. Results

### 2-1. Screening for senescent gene

For the purpose of searching for and identifying novel factor that control cellular senescence, a screening system was constructed. A system of inducing senescence by increasing the expression of the cell growth factor Ras protein in a drug-dependent manner in the normal human fibroblasts IMR90 (Innes et al., Methods Mol Biol. 1896: 83-92, 2019) was produced, and comprehensive gene knockdown was then performed on these cells using an siRNA library. At that time, a change in cellular senescence was evaluated by a fluctuation in the expression level of the cellular senescence marker p15 mRNA, and the genes whose cellular senescence was changed by knockdown were narrowed down. Among the narrowed down factors, Neurotrimin (NTM), which encodes a secretory protein and is expected to act like an SASP factor, attracted attention.

NTM is one type of GPI-anchored protein that is anchored to the cell membrane by glycosylphosphatidylinositol (GPI). NTM has a signal peptide sequence necessary for extracellular secretion at the N-terminus thereof, a signal peptide sequence necessary for GPI attachment at the C-terminus thereof, and further, three IgG-like domains in the center of the molecule thereof (see Figure 1B).

### 2-2. Knockdown of the NTM gene by siRNA

When NTM was knocked down by siRNA in IMR90 cells, normal human fibroblasts, (Figure 2A), an increase in the expression of p15 and IL-6 induced by Ras was suppressed (Figures 2B and C). Therefore, NTM was considered to have a function of inducing and/or promoting cellular senescence.

### 2-3. Forced expression of NTM

NTM was allowed to forcibly express in IMR90 cells using a retrovirus. As a result, an increase in the expression of p15 and IL-6 induced by Ras was promoted by the expression of NTM (Figures 3A and B). These results also suggest that NTM has a function of inducing and/or promoting cellular senescence.

### 2-4. Functional analysis of NTM

From the aforementioned results, it was anticipated that NTM secreted from cells acts on surrounding cells to induce senescence. Therefore, HeLa cells forcibly expressing NTM and IMR90 cells in which senescence was induced by Ras were co-cultured in a Transwell (see Figure 4A). If secreted NTM acts on cells to induce senescence, it is anticipated that NTM secreted from the HeLa cells will pass through the membrane and will act on the IMR90 cells to induce senescence. When the expression level of p15 serving as a senescence marker for the IMR90 cells co-cultured with the HeLa cells forcibly expressing NTM was examined, an increase in the expression was confirmed (Figure 4B). Therefore, it is considered that NTM secreted from the HeLa cells induced senescence in the IMR90 cells.

### 2-5. Induction of senescence by recombinant NTM

Next, a recombinant NTM protein (rNTM) was produced using *Escherichia coli,* and was then allowed to act on IMR90 cells in which senescence was induced by Ras. As a result, it was revealed that the expression of p15 and IL-6 was induced when rNTM (40 ng/mL) was added to the medium (Figures 5B and C). In addition, the expression of NTM genes was also induced (Figure 5A).

### 2-6. Role of NTM in senescence of mouse MEFs

Next, an analysis was performed using MEFs (mouse embryonic fibroblasts) prepared from fetuses of NTM-deficient mice. It is known that cellular senescence is caused by repeating the operation of passaging MEFs, and that cell proliferation is gradually reduced. Therefore, the operation of passaging MEFs derived from littermate wild-type mice and NTM-deficient mice was repeatedly carried out, and a change in proliferation was examined. As a result, it was revealed that a decrease in cell proliferation associated with an increase in the passage number was suppressed by NTM deficiency (Figures 6A and B). Furthermore, it was revealed that induction of the expression of p15, p16, and IL-6 associated with an increase in the passage number was suppressed by NTM deficiency (Figures 7A, B, and C).

From the aforementioned results, it was demonstrated that NTM has a function of inducing senescence even in mouse MEFs.

### 2-7. Role of NTM in obesity

Whether NTM is involved in senescence in mouse individuals was examined. Obesity is one of the methods of inducing senescence to mouse individuals. It is known that obesity causes chronic inflammation mainly in adipose tissues, which induces senescence. Thus, a change in the expression of NTM and senescence-related factors in the adipose tissues of db/db mice (which lack the receptor for the satiety hormone leptin), which are used as overeating-type obesity models, was examined. As a result, it was confirmed that the expression of p15, p16, and IL-6 was induced in adipose tissues due to obesity, and it was revealed that, at that time, the expression of NTM also showed a tendency to increase (Figure 8).

### 2-8. Suppression of NTM expression by calorie restriction

As an experiment opposite to obesity, a calorie restriction experiment was carried out using mice. It has been reported that calorie restriction is one of the most well-known methods of extending lifespan, and that calorie restriction suppresses senescence and extends lifespan in mice and higher organisms such as monkeys (Colman et al., Science 325: 201-204, 2009).
Thus, 40% calorie restriction (CR) was performed on mice for 30 weeks, and thereafter, a change in the expression of NTM and senescence-related factors in metabolic control organs was compared with that in a free-feeding group (Fed ad libitum; lib).

The body weight of mice was reduced by calorie restriction (Figure 9). The expression of the senescence-related factors p15 (Figure 10A), IL-6 (Figure 10B) and p16 (Figure 10C) in visceral adipose tissues (epididymal fat; epi) and subcutaneous adipose tissues (subcutaneous fat; sub) was decreased by calorie restriction, and suppression of senescence was clearly confirmed. It was confirmed that, at this time, the expression of NTM was significantly decreased mainly in adipose tissues (Figure 10D).

### 2-9. Analysis of NTM-deficient mice fed with high-fat diet

Under normal rearing conditions, female and male NTM-deficient mice showed no difference in body weight and insulin sensitivity, compared to female and male wild-type mice, when the NTM-deficient mice were young. Thus, in order to physiologically induce senescence, the NTM-deficient mice were fed with a high-fat diet to induce an obese state, and changes in body weight and insulin sensitivity that occurred during this process were then examined using an ITT (insulin tolerance test). ITT is an experiment in which insulin is administered to mice and the blood glucose levels thereof are then monitored over time. Mice with high insulin sensitivity show a rapid decrease in blood glucose levels after insulin administration, but mice that have acquired insulin resistance due to obesity, the decrease in blood glucose levels due to insulin administration is slow.

As a result, the NTM-deficient mice fed with a high-fat diet showed a tendency toward a decrease in body weight (Figure 11A), although the difference was not considered to be a significant difference (Figure 11B), and insulin sensitivity was significantly improved (Figure 11B). In addition, it also became clear that fasting blood glucose levels tended to decrease (Figure 12A), and that the value of the diabetes marker HbA1c was significantly decreased (Figure 12B). Furthermore, it was revealed that the expression of cellular senescence markers such as p15, p16, and p21 (Figure 13) and inflammatory markers (Figure 14) was decreased in the metabolic control organs, mainly adipose tissues.

### 2-10. Analysis using antibodies that inhibit NTM function

From the previous results, it became clear that 1) NTM induces senescence in other cells by being secreted outside the cells, or membrane-bound NTM induces senescence in cells expressing the NTM or in other adjacent cells, that 2) NTM is induced to be expressed in adipose tissues due to obesity, and that 3) the deficiency of NTM inhibits acquisition of insulin resistance in obesity. Based on these facts, it is considered that neutralizing antibodies against NTM (i.e., antibodies that inhibit the function of NTM) can be therapeutic drugs for various diseases such as cellular senescence, individual aging, inflammation, obesity/diabetes, and cancer. Thus, production of neutralizing antibodies against NTM has been attempted using fully human antibody-producing mice (i.e., mice that stably retain the full-length human antibody gene by chromosomal introduction) (contracted to Trans Chromosomics). Mice were immunized with cells in which NTM had been forcibly expressed, or with a recombinant NTM protein, and hybridoma lines that produce human monoclonal antibodies against NTM were established. Among the obtained clones, those having NTM-neutralizing activity, namely, clones having a function of inhibiting induction of senescence were selected using suppression of the expression of the p15 gene and IL-6 gene as an indicator. As a result, antibodies capable of suppressing the expression of p15 and IL-6 in IMR90 cells, to which senescence was induced by Ras, were successfully obtained (the aforementioned antibodies (A) to (E)).

Specifically, the obtained anti-NTM human monoclonal antibody (the aforementioned antibody (A)) was added to the culture medium of IMR90 cells to a concentration of 100 ng/mL or 1,000 ng/mL, the IMR90 cells were then cultured for 4 days with the culture medium, and the expression levels of the p15 and IL-6 genes in the IMR90 cells were then measured. As a result, it was revealed that the expression levels of the p15 and IL-6 genes in the IMR90 cells were suppressed by the addition of the antibody (Figure 15).

### 2-11. Studies of influence of NTM on decrease in muscle strength due to aging

### 2-11-1. Decrease in muscle strength due to aging

In order to study the influence of aging on muscle strength, a grip strength test was performed. The muscle strength of 180-day-old young mice (male and female) and 690-day-old old mice was measured and compared. As a result, it was confirmed that aging causes a decrease in the muscle strength in both male and female mice (Figure 16, male: left figure, female: right figure).

### 2-11-2. Effects of NTM on decrease in muscle strength due to aging

A grip strength test was performed to compare the muscle strength of 600-day-old senescent wild-type mice with the muscle strength of NTM-deficient mice. As a result, it was found that NTM deficiency significantly suppressed a decrease in the muscle strength in the old female and male mice (Figure 17, male: left figure, female: right figure).

From these results, it was conceived that NTM deficiency has the effect of suppressing a decrease in the muscle strength in old mice.

### 2-12. Studies of influence of NTM on decrease in muscle endurance due to aging

### 2-12-1. Decrease in muscle endurance due to aging

A hanging test was performed to examine the influence of aging on muscle endurance. The muscle endurance of 180-day-old young female mice and 690-day-old old female mice was measured and compared. With regard to both the hanging time and the hanging score, the values of the old mice were lower than those of the young mice, and thus, it was confirmed that the muscle endurance is decreased by aging (Figure 18, Hanging time: left figure, Hanging score: right figure).

### 2-12-2. Effects of NTM on decrease in muscle endurance due to aging

A hanging test was performed to compare the muscle endurance of 600-day-old aged wild-type mice with the muscle endurance of NTM-deficient mice. As a result, it was found that a decrease in the muscle endurance in the old female and male mice was significantly suppressed by NTM deficiency (Figure 19: Results for male mice, Figure 20: Results for female mice).

From these results, it was conceived that NTM deficiency has the effect of suppressing a decrease in the muscle endurance in old mice.

### 2-13. Calculation and analysis of frailty scores in old NTM-deficient mice

In order to evaluate the influence of NTM on aging in individual mice using a more comprehensive indicator based on the senescence phenotype, the frailty scores of 960-day-old aged wild-type male mice and NTM-deficient male mice were calculated. As a result, it was found that NTM deficiency tends to suppress progression of frailty syndrome in aged mice (Figure 22).

From these results, it was conceived that NTM deficiency suppresses a decrease in the muscle strength and the muscle endurance, as well as suppressing aging in individual mice.

From the aforementioned experimental results, it was suggested that cellular senescence is inhibited or suppressed by inhibiting NTM, and that it is possible to suppress individual aging and to prevent and treat diseases developed by aging.

### Industrial Applicability

The present invention provides a medicament or the like that is useful for the improvement of aging and obesity, and for prevention or treatment of diseases, etc. developed with aging. Therefore, it is expected that the present invention will be utilized in medical field.

## Claims

1. A cellular senescence-suppressing agent, comprising, as an active ingredient, an NTM (Neurotrimin) function inhibitor.

2. The cellular senescence-suppressing agent according to claim 1, wherein the NTM function inhibitor is an antibody, a peptide aptamer, a specialty peptide, a nucleic acid or a low-molecular-weight compound.

3. An agent for improving individual aging, comprising the cellular senescence-suppressing agent according to claim 1 or 2.

4. An agent for improving obesity, comprising the cellular senescence-suppressing agent according to claim 1 or 2.

5. A pharmaceutical composition for preventing or treating diseases provoked by cellular senescence, the pharmaceutical composition comprising the cellular senescence-suppressing agent according to claim 1 or 2.

6. The pharmaceutical composition according to claim 5, wherein the diseases are cancer, diabetes, hypertension, dyslipidemia, arteriosclerosis, cerebral infarction, heart disease (myocardial infarction, cardiomyopathy and cardiac hypertrophy), chronic obstructive pulmonary disease, chronic kidney disease, chronic liver disease (cirrhosis, hepatitis and fatty liver), dementia (Alzheimer's disease), Parkinson's disease, frailty syndrome, sarcopenia, emaciation, osteoarthritis, spondylosis deformans, skin disease, senile alopecia, cataracts, dry eyes, glaucoma, age-related macular degeneration, presbyopia, other chronic inflammatory diseases, and other metabolic diseases and/or infectious diseases.

7. A food and drink composition or a cosmetic composition, comprising the cellular senescence-suppressing agent according to claim 1 or 2.

8. The food and drink composition or the cosmetic composition according to claim 7, which is for use in anti-aging, dieting, or lowering blood sugar levels.

9. An antibody, which is **characterized in that** the amino acid sequences of CDRs (complementarity determining regions) 1 to 3 satisfy any of the following (A), (B), (C), (D) or (E), or an antigen-binding fragment thereof:
(A)
heavy chain CDR1 comprising the amino acid sequence as set forth in SEQ ID No: 3,
heavy chain CDR2 comprising the amino acid sequence as set forth in SEQ ID No: 4,
heavy chain CDR3 comprising the amino acid sequence as set forth in SEQ ID No: 5,
light chain CDR1 comprising the amino acid sequence as set forth in SEQ ID No: 6,
light chain CDR2 comprising the amino acid sequence as set forth in SEQ ID No: 7, and
light chain CDR3 comprising the amino acid sequence as set forth in SEQ ID No: 8;
(B)
heavy chain CDR1 comprising the amino acid sequence as set forth in SEQ ID No: 9,
heavy chain CDR2 comprising the amino acid sequence as set forth in SEQ ID No: 10,
heavy chain CDR3 comprising the amino acid sequence as set forth in SEQ ID No: 11,
light chain CDR1 comprising the amino acid sequence as set forth in SEQ ID No: 12,
light chain CDR2 comprising the amino acid sequence as set forth in SEQ ID No: 13, and
light chain CDR3 comprising the amino acid sequence as set forth in SEQ ID No: 14;
(C)
heavy chain CDR1 comprising the amino acid sequence as set forth in SEQ ID No: 15,
heavy chain CDR2 comprising the amino acid sequence as set forth in SEQ ID No: 16,
heavy chain CDR3 comprising the amino acid sequence as set forth in SEQ ID No: 17,
light chain CDR1 comprising the amino acid sequence as set forth in SEQ ID No: 18,
light chain CDR2 comprising the amino acid sequence as set forth in SEQ ID No: 19, and
light chain CDR3 comprising the amino acid sequence as set forth in SEQ ID No: 20;
(D)
heavy chain CDR1 comprising the amino acid sequence as set forth in SEQ ID No: 21,
heavy chain CDR2 comprising the amino acid sequence as set forth in SEQ ID No: 22,
heavy chain CDR3 comprising the amino acid sequence as set forth in SEQ ID No: 23,
light chain CDR1 comprising the amino acid sequence as set forth in SEQ ID No: 24,
light chain CDR2 comprising the amino acid sequence as set forth in SEQ ID No: 25, and
light chain CDR3 comprising the amino acid sequence as set forth in SEQ ID No: 26; or
(E)
heavy chain CDR1 comprising the amino acid sequence as set forth in SEQ ID No: 27,
heavy chain CDR2 comprising the amino acid sequence as set forth in SEQ ID No: 28,
heavy chain CDR3 comprising the amino acid sequence as set forth in SEQ ID No: 29,
light chain CDR1 comprising the amino acid sequence as set forth in SEQ ID No: 30,
light chain CDR2 comprising the amino acid sequence as set forth in SEQ ID No: 31, and
light chain CDR3 comprising the amino acid sequence as set forth in SEQ ID No: 32.

10. An antibody that suppresses or inhibits the function of NTM, the antibody competitively inhibiting the binding between the antibody according to claim 9 and NTM, or an antigen-binding fragment thereof.

11. The antigen-binding fragment according to claim 9 or claim 10, which is **characterized in that** it is Fab, Fab', F (ab')₂, Fv, a single-chain antibody, scFv, an scFv dimer, or dsFv.

12. A method of screening for a substance that suppresses cellular senescence, comprising the following (a) and (b):
(a) a step of allowing NTM and a candidate substance to come into contact with cells; and
(b) a step of evaluating the degree of cellular senescence of the cells with which the NTM and the candidate substance are allowed to come into contact.

13. The method according to claim 12, wherein the degree of cellular senescence is evaluated using, as an indicator, the expression level of a cellular senescence marker or the expression level of an SASP (senescence-associated secretory phenotype)-related factor.

14. The method according to claim 13, wherein the cellular senescence marker is p16, p15, or p21.

15. The method according to claim 13, wherein the SASP-related factor is an IL-6 or IL-8 gene.
